# EUROPEAN PATENT APPLICATION

(11) **EP 4 560 012 A2**
(43) Date of publication of application: **28.05.2025**
(21) Application number: 25155602.3
(22) Date of filing: 10.04.2018
(51) Int. Cl.: C12N 5/0781

(54) **MOUSE MODELS AND THERAPEUTIC MOLECULES**

(30) Priority: 10.04.2017 GB 201705725
(62) Divisional of application: 18719629.0
(71) Applicant: Genome Research Limited, Hinxton, Cambridge CB10 1SA (GB)
(72) Inventor: MARTIN, Jolyon Nicolas Edouard, Cambridge CB10 1SA (GB); BRADLEY, Allan, Cambridge CB10 1SA (GB)
(74) Representative: CMS Cameron McKenna Nabarro Olswang LLP

(57) **Abstract**

The present invention relates inter alia to a rodent or rodent cell having a genome comprising: i) one or more companion animal IGH V region genes, one or more companion animal D region genes and one or more companion animal J region genes; and(ii) optionally one or more companion animal IGL kappa V region genes and one or more companion animal IGL kappa J region genes; and/or one or more companion animal IGL lambda V region genes and one or more companion animal IGL lambda J region genes, wherein the rodent or rodent cell is capable of expressing the companion animal variable region gene(s) to form an antibody chain and wherein the companion animal species is not a rodent.

## Description

### Background

The present invention relates inter alia to rodents and cells that are engineered to contain the exogenous DNA of companion animals, their use in medicine and the study of disease, methods for production of rodents and cells, and antibodies and antibody chains produced by such animals and derivatives thereof.

Insertion of human DNA into rodents has been disclosed in eg Murphy et al, Vol 111 no 14, 5153-5158, doi: 10.1073/pnas.1324022111; MacDonald et al vol. 111 no. 14, 5147-5152, doi: 10.1073/pnas.1323896111; and Lee et al , Nature Biotechnology Volume: 32, Pages:356-363Year published:, 2014DOI:, doi:10.1038/nbt.2825. This approach is designed to make antibody products for human therapeutic use. However, no rodent models have been developed to generate antibodies suitable for use in other species, such as companion animals.

The present invention relates to rodents, cells, antibodies and parts thereof produced therefrom, including subsequently modified antibodies which are for use in companion animals, as well as methods for the manufacture of such rodents, cells, antibodies and antibody chains.

### Statements of invention

### The invention relates to

A rodent or rodent cell having a genome comprising;
i) one or more companion animal IGH V region genes, one or more companion animal IGH D region genes and one or more companion animal IGH J region genes; and
ii) optionally one or more companion animal IGL kappa V region genes and one or more companion animal IGL kappa J region genes; and/or one or more companion animal IGL lambda V region genes and one or more companion animal IGL lambda J region genes

wherein the rodent or rodent cell is capable of expressing the companion animal variable region gene(s) to form an antibody chain
and wherein the companion animal species is not a rodent.

A rodent or rodent cell having a genome comprising
i) one or more companion animal IGL kappa V region genes and one or more companion animal IGL kappa J region genes; and/or one or more companion animal IGL lambda V region genes and one or more companion animal IGL lambda J region genes, and
ii) optionally one or more companion animal IGH V region genes, one or more companion animal or host IGH D region genes and one or more companion animal or host IGH J region genes

wherein the rodent or rodent cell is capable of expressing the companion animal variable region gene(s) to form an antibody chain
and wherein the companion animal species is not a rodent.

A method for producing a rodent or rodent cell, the method comprising inserting into a rodent cell genome either
i) one or more companion animal IGH V region genes, one or more companion animal IGH D region genes and one or more companion animal IGH J region genes; and/or
ii) one or more companion animal IGL kappa V region genes and one or more companion animal IGL kappa J region genes; and/or
one or more companion animal IGL lambda V region genes and one or more companion animal IGL lambda J region genes
wherein the rodent or rodent cell is capable of expressing the companion animal gene(s) to form an antibody chain in combination with a constant region of the rodent or companion animal.

A method for producing an antibody chain or antibody specific to a desired antigen, the method comprising immunizing a rodent as disclosed herein with the desired antigen and recovering the antibody chain, singularly or as part of a complete antibody, or recovering a cell producing the antibody chain singularly or as part of a complete antibody.

A method for producing an antibody chain or antibody specific to a desired antigen and derived from a single species of companion animal, the method comprising immunizing a rodent comprising companion animal genes as disclosed herein and then replacing the rodent constant region of the antibody chain with a companion animal constant region from the same companion animal, suitably by engineering of the nucleic acid encoding the antibody chain or antibody.

A method for making an antibody, or part thereof, the method comprising providing:
(i) a nucleic acid encoding an antibody, or a part thereof, obtained according to the present invention; or
(ii) sequence information from which a nucleic acid encoding an antibody obtained according to the present invention, or part thereof, can be expressed to allow an antibody to be produced

A method for producing an antibody chain or part thereof, the antibody chain having a companion animal variable region, the method comprising expressing in a cell a nucleic acid encoding the antibody chain, or a part thereof,
wherein the sequence of the DNA encoding the variable region of the antibody chain is obtained or obtainable by immunising a rodent of the invention with an antigen so that antibody chains are produced,
optionally including the subsequent steps of:
   purifying and /or isolating the antigen receptor chain, and
   optionally then formulating the antigen receptor chain into a pharmaceutically acceptable formulation suitable for administration into a companion animal, preferably the same companion animal as the variable region, or into a human or other mammal in need thereof.

An antibody or antibody chain, or part thereof, obtained or obtainable from a rodent or cell according to the present invention.

An antibody or antibody or chain, or part thereof, obtained or obtainable according to the invention, for use in treatment of a companion animal.

A method of treatment of a companion animal, the method comprising delivery of an antibody or antibody chain or part thereof to a companion animal in need thereof, wherein the antibody or antibody chain or part thereof has been modified to be a fully companion animal antibody.

### Figures

Figure 1 - The canine immunoglobulin heavy chain locus, overlaid with bacterial artificial chromosomes spanning the locus
Figure 2 - The canine immunoglobulin kappa locus, overlaid with bacterial artificial chromosomes spanning the locus
Figure 3 - The canine immunoglobulin lambda locus, overlaid with bacterial artificial chromosomes spanning the locus
Figure 4 - The feline immunoglobulin heavy chain locus, overlaid with bacterial artificial chromosomes spanning the locus
Figure 5 - The feline immunoglobulin kappa locus, overlaid with bacterial artificial chromosomes spanning the locus
Figure 6 - The feline immunoglobulin lambda locus, overlaid with bacterial artificial chromosomes spanning the locus
   BACs are shown in Figures 1-6 to further illustrate both how groups of canine and feline genes are located in publicly available BACs, and how such genes may then be incorporated into rodent loci according to the present invention.
Figure 7 - Allele changes. The prevalence of non-reference V gene alleles is plotted per breed based on three classes. Alleles that were functional where the reference allele was not, as well as those that were ORFs where the reference was a pseudogene, are grouped as 'Gain'. Alleles that were pseudogenes where the reference allele was not, as well as those that were functional and became ORFs, are grouped as 'Loss'. Alleles with no functionality change are classed as 'None'. Black crosshairs represent the expected values for each change type.
Figure 8 - Self-alignment of the canine immunoglobulin kappa locus. The green box (A) represents the upstream V genes aligned to themselves. The red box (C) represents the downstream V genes aligned to themselves. The blue box (B) represents the alignment of the upstream V genes against the downstream ones.
Figure 9 - The alignment of the canine and human immunoglobulin loci. (A) The complete loci. (B) An amplified section of the alignment, corresponding to the red box in (A).
Figure 10 - Canine BAC 1 insertion into the murine IGH locus

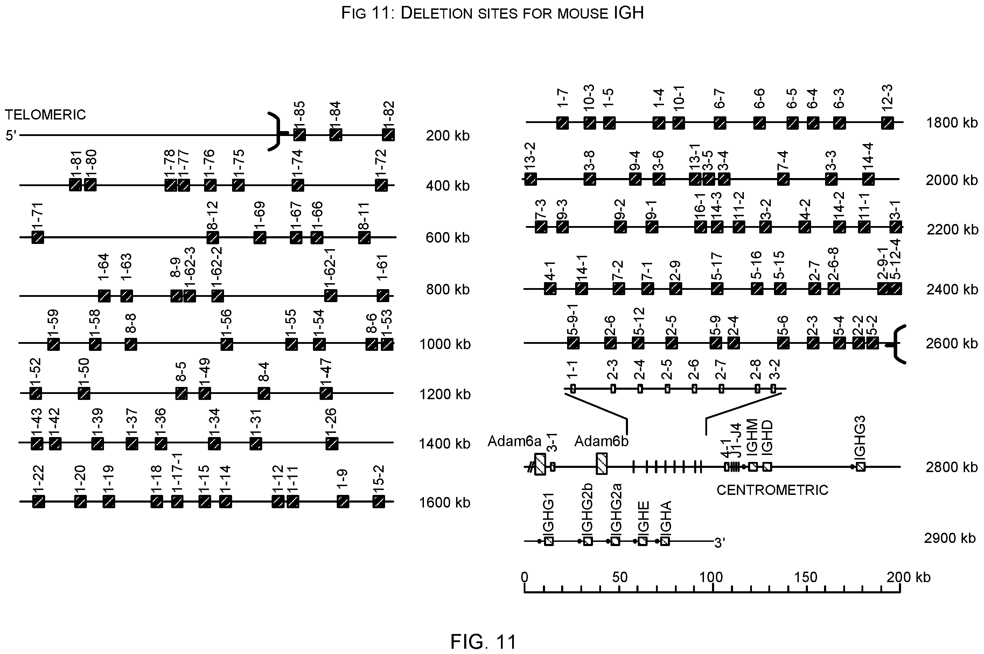

Figure 11 - Deletion sites for mouse IGH

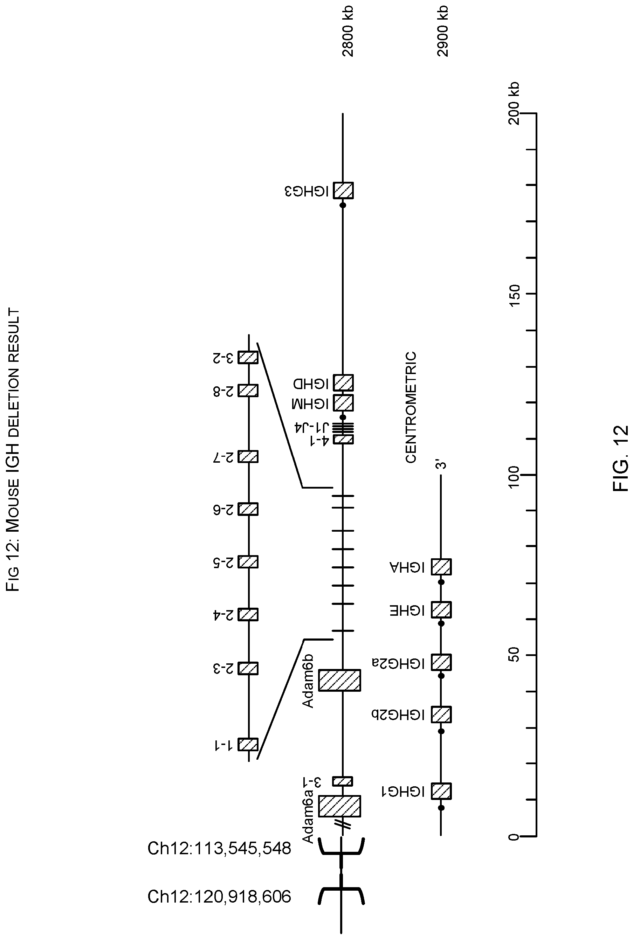

Figure 12 - Mouse IGH deletion result
Figure 13 - Canine BAC 1 insertion into the murine IGL lambda locus

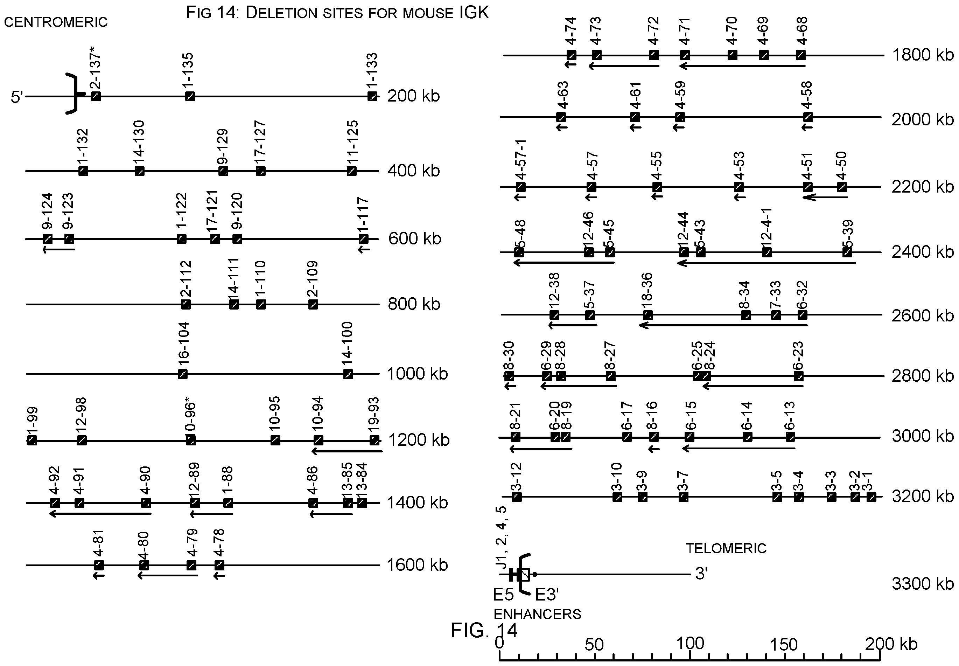

Figure 14 - Deletion sites for mouse IGK
Figure 15 -Expression of chimaeric transcripts confirmed by PCR analysis from the chimaeric heavy chain locus
Fig 16: Strong germline Canine-Human IG Homology, TCR homology is less conserved

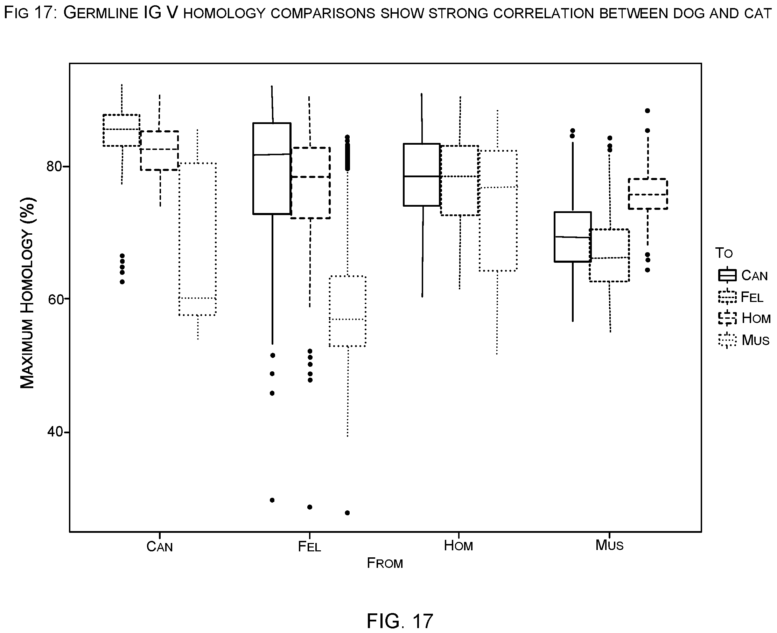

Fig 17: Germline IG V homology comparisons show strong correlation between dog and cat

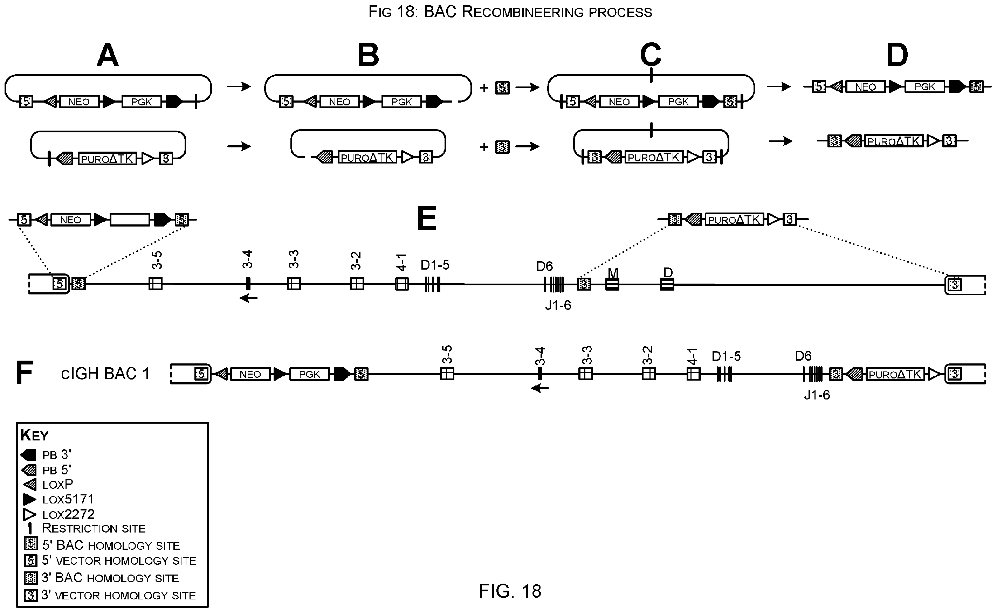

Figure 18 - BAC Recombineering process. A: unmodified 5' (top) and 3' (bottom) modifying vectors with linearisation site shown in red. B: linearised vectors with the BAC homology arms, ready for Gibson assembly. C: Assembled vectors ready for digestion to release vector fragment. Digestion sites shown in red. D: purified vector fragments ready for recombineering. E: Recombineering takes place along dashed lines between fragments and unmodified BAC. F: Recombineered BAC ready for S-RMCE.

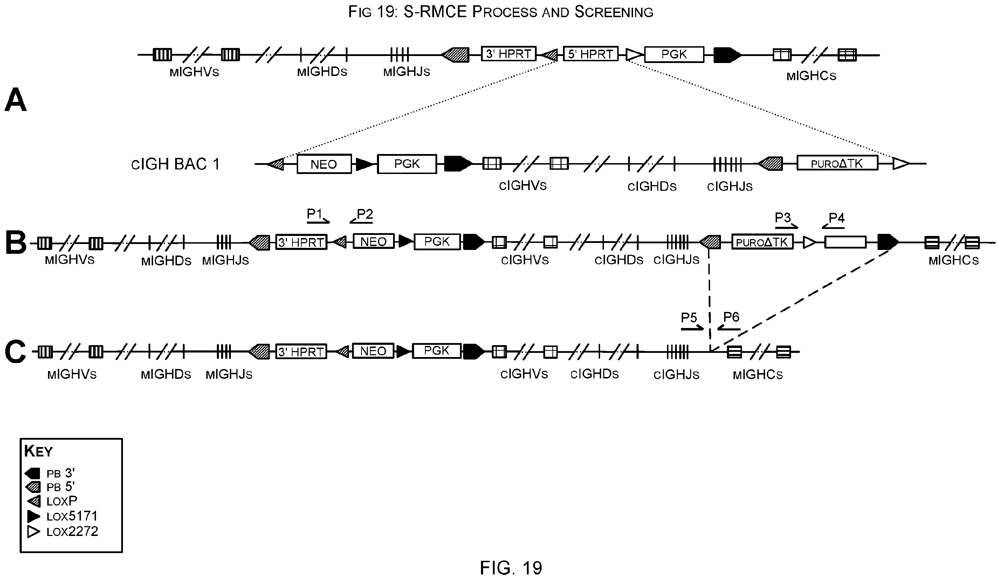

Figure 19: S-RMCE process and screening. A: Recombineered BACs are inserted into the landing pad through the action of Cre-recombinase. B: Successful BAC insertion can be screened for using primer pairs P1 + P2 and P3 + P4. C: Excision of the 3' modifying DNA through the action of PBase (excision points indicated by dashed lines). Successful excision can be screened for with primer pair P5 + P6.

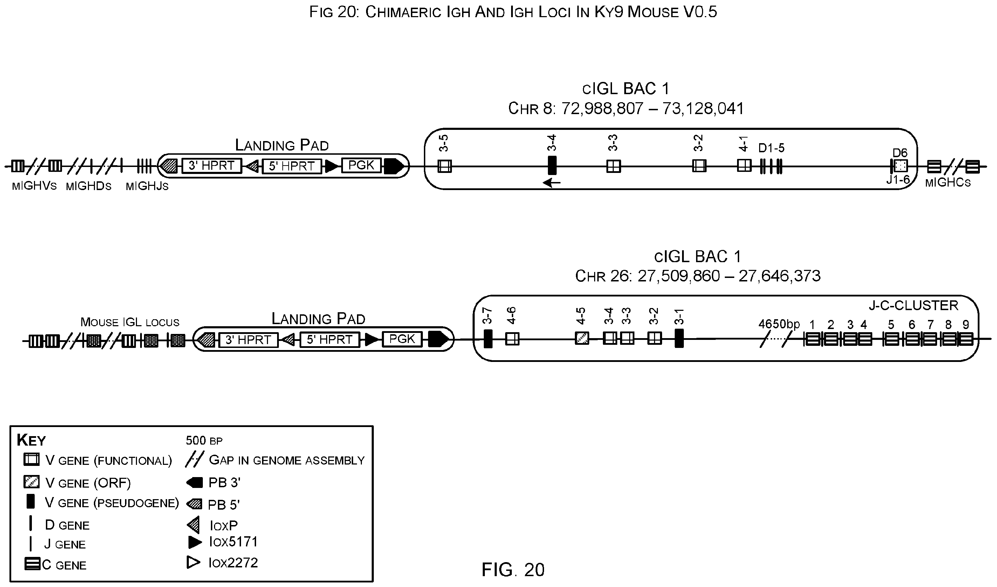

Fig 20: chimaeric IGH and IGL loci in Ky9 mouse V0.5
Fig 21: Ky9 mouse V0.5 produces highly diverse antibody repertoires

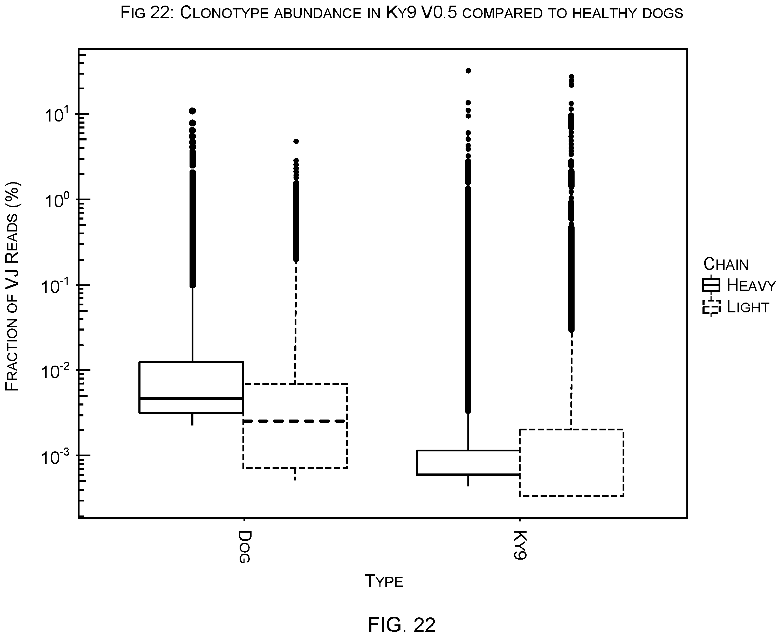

Fig 22: Clonotype abundance in Ky9 V0.5 compared to healthy dogs

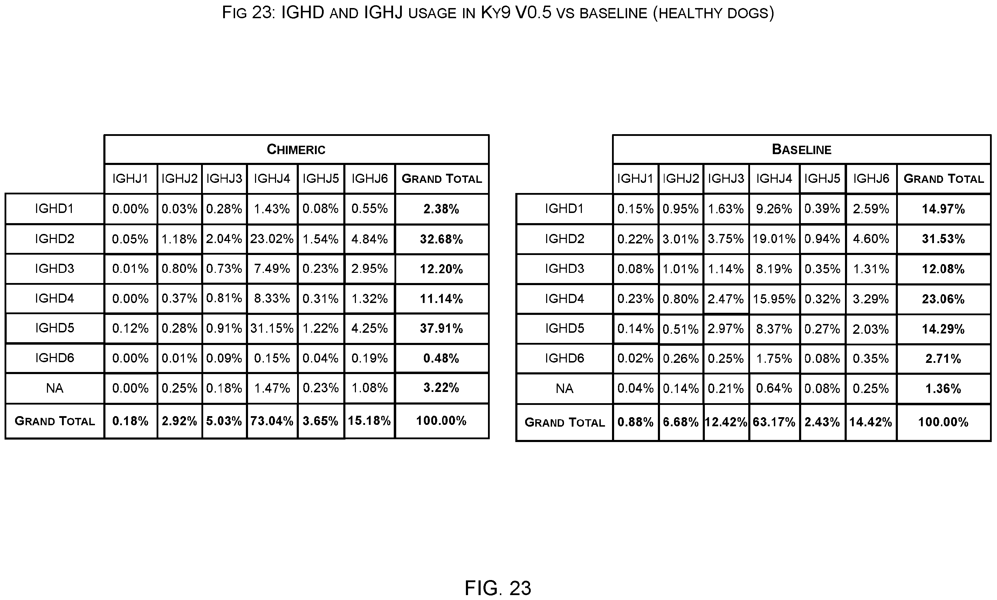

Fig 23: IGHD and IGHJ usage in Ky9 V0.5 vs baseline (healthy dogs)

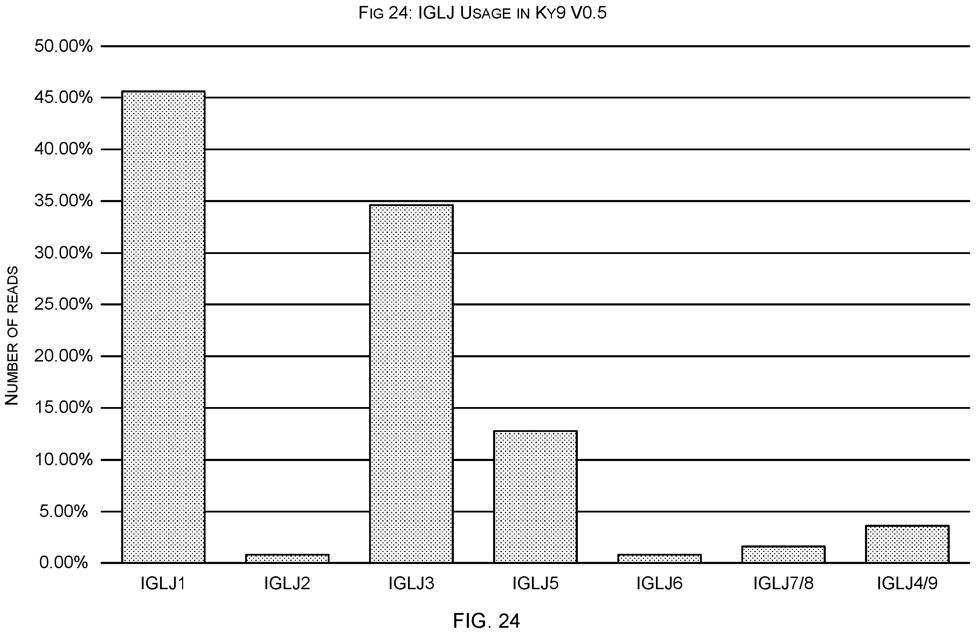

Fig 24: IGLJ Usage in Ky9 V0.5

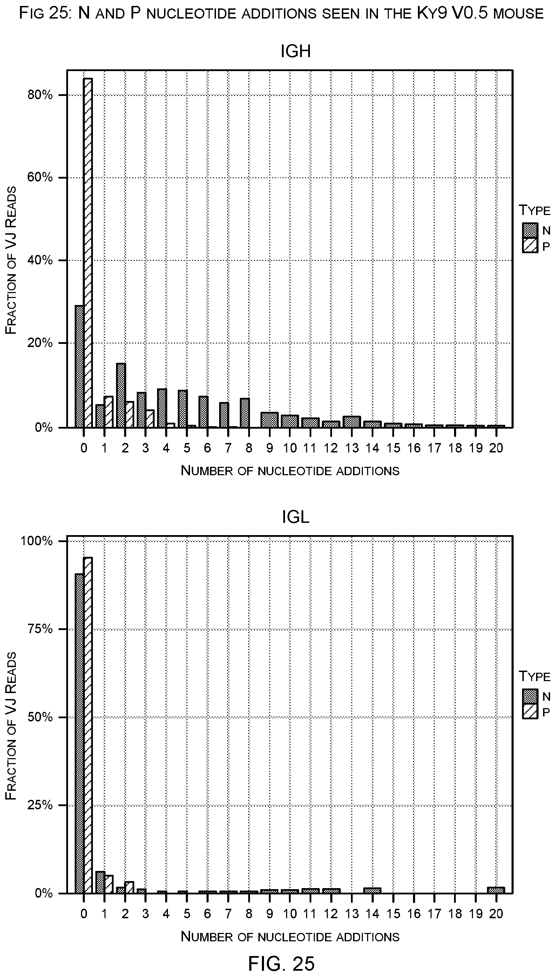

Fig 25: N and P nucleotide additions seen in the Ky9 V0.5 mouse

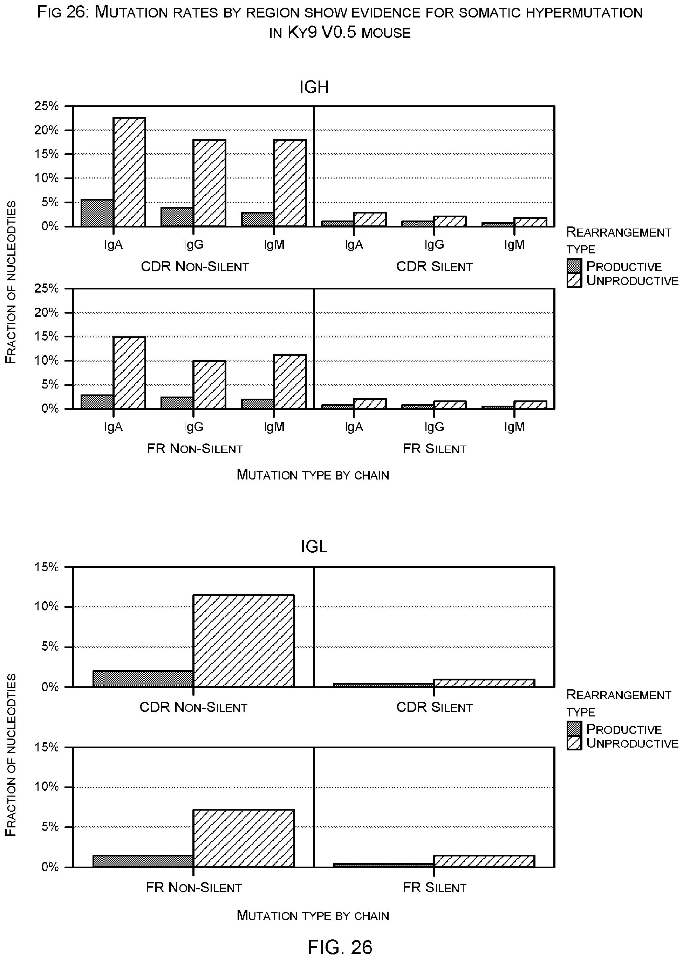

Fig 26: Mutation rates by region show evidence for somatic hypermutation in Ky9 V0.5 mouse

### Detailed description

The present invention relates to a rodent or rodent cell having a genome comprising;
i) one or more companion animal IGH V region genes, one or more companion animal IGH D region genes and one or more companion animal IGH J region genes;
ii) optionally comprising one or more companion animal IGL kappa V region genes and one or more companion animal IGL kappa J region genes; and/or one or more companion animal IGL lambda V region genes and one or more companion animal IGL lambda J region genes

wherein the rodent or rodent cell is capable of expressing the companion animal variable region gene to form an antibody chain in combination with an antibody constant region
and wherein the companion animal species is not a rodent

### The invention also relates to:

A rodent or rodent cell having a genome comprising
i) one or more companion animal IGL kappa V region genes and one or more companion animal IGL kappa J region genes; and/or one or more companion animal IGL lambda V region genes and one or more companion animal IGL lambda J region genes, and
ii) optionally comprising one or more companion animal IGH V region genes, one or more companion animal IGH D region genes and one or more companion animal IGH J region genes

wherein the rodent or rodent cell is capable of expressing the companion animal variable region gene(s) to form an antibody chain in combination with an antibody constant region
and wherein the companion animal species is not a rodent.

By way of illustrative example, not binding on the invention, the insertion of immunoglobulin heavy (IGH) chain variable (V) region genes, IGH D region genes and IGH J region genes from a dog into a mouse allows the production of antibody heavy chains that comprise a variable antibody region originating from the expression of canine DNA in the mouse, in combination with a constant region. The constant region may be the rodent immunoglobulin (IG) constant region, resulting in production of chimaeric heavy chains having canine variable region and a rodent constant region. Information concerning, or the nucleic acid comprising, the variable region of such chimaeric antibody chains may be used to generate fully canine antibodies, for therapeutic use in dogs for example. The rodent containing the canine DNA may also serve as an animal model for understanding of disease and testing of medicines.

All nucleotide co-ordinates for the mouse are those corresponding to the Dec 2011 GRCm38/mm10 assembly for the mouse (assembly accession GCA_000001635.2) - unless otherwise specified.

For the avoidance of doubt, the insertion points referenced in the mouse genome are identical to those detailed in Lee et al, Nature Biotechnology, Nature Biotechnology 32,356-363(2014).

Dog genome build is CanFam3.1 (assembly accession - GCA_000002285.2), produced September 2011 and last updated May 2016.

Cat genome build is FelisCatus8.0 (assembly accession - GCA_000181335.3), produced November 2014.

The rodent of the invention is preferably a mouse or rat, and is preferably a mouse.

Companion animals of the invention are suitably selected from dogs, cats, horses, birds, rabbits, goats, reptiles, fish and amphibians. A dog is a preferred companion animal of the invention. A cat is a preferred companion animal of the invention. A horse is a preferred companion animal of the invention. For the avoidance of doubt a human is not a companion animal.

In one aspect the rodent is a mouse and the companion animal is a dog.

In one aspect the rodent is a mouse and the companion animal is a cat.

In one aspect the rodent is a mouse and the companion animal is a horse.

The IG heavy (IGH) genomic locus from companion animals comprises multiple V, D and J region genes. Expression of a V, a D and a J region gene together produces the variable region of the heavy chain of an antibody. IGH V, D and J genes are naturally expressed in combination with a heavy chain constant region. The IG light chain locus (IGL) - which may be lambda or kappa, comprises multiple V and J gene segments which, when expressed together, form the variable region of the light chain of the antibody. IGL V and J region genes are naturally expressed in combination with a light chain constant region of the kappa or lambda light chain. The rodent or rodent cell of the invention is capable of expressing the companion animal VDJ or VJ region gene(s) to form an antibody chain. The companion animal genes are operably linked in the rodent genome with a constant region to allow an antibody chain to be expressed. The companion animal IG genes may be located in the rodent genome along with an exogenous constant region gene (from a species other than the rodent), or may be located in the rodent genome in functional arrangement with, such as upstream of, a rodent constant region present naturally in the rodent genome, such that expression of the V region genes with the constant region can occur.

The rodent or rodent cell genome may comprise one or more companion animal IGH V and IGH D and IGH J region genes but no light chain companion animal DNA, or may comprise one or more companion animal IGL V and IGL J region genes but no heavy chain companion animal DNA. The rodent or cell genome may also comprise companion animal genes from the heavy and kappa chains (but not lambda), or heavy and lambda chains (but not kappa), or comprise companion animal genes from all three loci, heavy, kappa and lambda.

In one aspect the inserted companion animal DNA comprises at least 50% of the companion animal heavy chain variable (V) genes, such as at least 60%, at least 70%, at least 80%, at least 90%, and in one aspect all of the companion animal V genes.

In one aspect the inserted companion animal DNA comprises at least 50% of the companion animal heavy chain diversity (D) genes, such as at least 60%, at least 70%, at least 80%, at least 90%, and in one aspect all of the companion animal D genes.

In one aspect the inserted companion animal DNA comprises at least 50% of the companion animal heavy chain joining (J) genes, such as at least 60%, at least 70%, at least 80%, at least 90%, and in one aspect all of the companion animal J genes.

In one aspect the inserted companion animal DNA comprises at least 50% of the companion animal light chain variable (V) genes, such as at least 60%, at least 70%, at least 80%, at least 90%, and in one aspect all of the companion animal light chain V genes.

In one aspect the inserted companion animal DNA comprises at least 50% of the companion animal light chain joining (J) genes, such as at least 60%, at least 70%, at least 80%, at least 90%, and in one aspect all of the companion animal light chain J genes.

In one aspect the rodent genome comprises all of the IGH V, D and J region genes and intervening sequences from the companion animal.

In one aspect the rodent genome comprises all of the IGL kappa V and J region genes and intervening sequences from the companion animal.

In one aspect the rodent genome comprises all of the IGL lambda V and J region genes and intervening sequences from the companion animal.

The rodent or rodent cell genome may comprise at least 4, 5, 10, 15 or 20, companion animal IGH V region genes, such as at least 30, 40, 50, 60 70, 80 V region genes. In one preferred aspect these are canine V region genes. In one preferred aspect the rodent genome comprises at least 83 canine IG heavy chain V region genes.

The rodent or rodent cell genome may comprise at least 1, 2, 3, 4, 5 or 6 IGHD region genes from a companion animal, preferably canine genes.

The rodent or rodent cell genome may comprise at least 1, 2, 3, 4, 5 or 6 IGHJ region genes from a companion animal, preferably canine genes.

The rodent or rodent cell genome may comprise at least 10, 15, 16, 17, 18 or 19 companion animal IGL kappa V region genes. In a preferred aspect these are canine kappa V region genes. In one preferred aspect the rodent genome comprises at least 19 canine light chain kappa V region genes.

The rodent or rodent cell genome may comprise at least 1, 2, 3, 4, or 5 IGL kappa J region genes from a companion animal, preferably canine genes.

The rodent or rodent cell genome may comprise at least 5, 10, 20, 30, 40, 50, 60, 70, 80, 90, 100, 110, 120, 130, 140, 150, or at least 160 companion animal IGL lambda V region genes. In a preferred aspect these are canine lambda V region genes. In one preferred aspect the rodent genome comprises at least 160 canine light chain lambda V region genes.

The rodent or rodent cell genome may comprise at least 1, 2, 3, 4, 5, 6, 7, 8, or 9 IGL lambda J region genes from a companion animal, preferably canine genes.

In another aspect the rodent or rodent cell genome may comprise at least 4, 5, 10, 15 or 20, IGH V region genes, such as at least 23 V region genes, from a cat.

In another aspect the rodent or rodent cell genome may comprise at least 4, 5, 10, or 11 companion animal IGH D region genes from a cat.

In another aspect the rodent or rodent cell genome may comprise at least 1, 2, 3, 4 or 5 companion animal IGH J region genes from a cat.

In another aspect the rodent or rodent cell genome may comprise at least 4, 5, 10, 15 IGL kappa V region genes, from a cat.

In another aspect the rodent or rodent cell genome may comprise at least 1, 2, 3, 4, 5 or 6 companion animal IGH kappa J region genes from a cat.

In another aspect the rodent or rodent cell genome may comprise at least 5, 10, 15, 20, 30, 40, 50, 60, 70, 80, 90, 100, 100 or more, such as 113 IGL lambda V region genes, from a cat.

In another aspect the rodent or rodent cell genome may comprise at least 1, 2, 3, 4, 5, 6, 7, 8, or 9 companion animal IGH lambda J region genes from a cat.

The number of companion animal genes referred to above in any aspect may also be further increased, and in one aspect is doubled, in the case of a homozygote having an insertion at both alleles.

Preferably the V, D and J region genes that are inserted into the genome are from the same companion animal. Preferably the inserted IGH VDJ region genes, or the IGL VJ region genes, are all canine, or are all feline, or are all equine. Preferably the genes are all canine.

In one aspect the inserted companion animal genes are all from the same breed of companion animal, for example, from the same breed of dog.

In one aspect the companion animal gene(s) are located in the genome upstream of a rodent constant region, suitably located upstream of a heavy chain constant region or regions for inserted companion animal heavy chain variable region genes, and/or suitably located upstream of a light chain constant region for inserted companion animal light chain variable region genes, such that the rodent or rodent cell is able to produce a chimaeric antibody chain resulting from expression of the inserted variable region gene and the rodent constant region.

Preferably the heavy chain V, D and J region genes from the companion animal are located in the rodent genome upstream of the rodent heavy chain constant region.

Preferably the light chain kappa V, J region genes from the companion animal are located in the rodent genome upstream of the rodent kappa light chain constant region.

Any reference to the location of the variable region upstream of a constant region, such as the rodent constant region, means that there is a suitable relative location of the two genomic portions, encoding the variable and constant regions of the antibody, to allow a chimaeric antibody chain to be expressed *in vivo* in the rodent. In this way the inserted companion animal DNA and rodent constant region are in functional arrangement with one another for antibody or antibody chain production.

In one aspect the inserted companion animal DNA, such as the variable VDJ or VJ region gene(s) are located in the rodent genome at a site which is distinct from that of the naturally occurring heavy or light constant region, such as on a different chromosome. In this case the insertion of the VDJ or VJ region genes is accompanied by a constant region and preferably also by a 3' enhancer from the rodent or from the companion animal. One preferred embodiment is the use of a rodent constant region and rodent 3' enhancer with companion animal VDJ or VJ regions, such as a canine constant region and canine 3' enhancer. In one aspect the companion animal gene(s) are located in the genome in functional arrangement with a constant region from the same companion animal, such that the rodent is able to produce an antibody chain resulting from the expression of the inserted companion animal VDJ or VJ region genes and the companion animal constant region. Alternatively the companion animal gene(s) are located in the genome in functional arrangement with a constant region from something other the companion animal, such as a different companion animal, or such as a constant region from the rodent.

Where companion animal genes are inserted into the rodent genome in association with a constant region then it will be appreciated that the insertion may be at any suitable location in the genome of the rodent cell, and may not be targeted at a rodent IG locus, as the endogenous constant region genes are not necessary for antibody chain production. The insertion may be at a random location into the rodent genome.

The invention also specifically contemplates cells and rodents having an insertion of companion animal genes in association with a companion animal constant region (encoding a "fully" companion animal antibody chain) at an endogenous rodent IG locus, such as DNA encoding a companion animal lambda V and J and C genes.

Preferably the light chain lambda V and J region genes from the companion animal are located in the genome in functional arrangement with, such as upstream of, a lambda chain constant region from the same companion animal. In this way a lambda antibody chain is generated that has the lambda constant region from the companion animal. The invention therefore relates to a rodent or rodent cell wherein the genome comprises one or more companion animal IGL lambda V region genes, one or more companion animal IGL lambda J region genes and one or more companion animal lambda constant regions, and the cell or rodent can express lambda antibody chains having both companion animal variable and constant regions.

In one aspect the companion animal VJC lambda antibody chain described above is inserted into mouse lambda locus, preferably between last rodent C gene and 3' enhancer.

In one aspect the rodent or rodent cell comprises one or more companion animal IGL lambda V region genes, one or more companion animal IGL lambda J region genes and one or more companion animal lambda constant regions located at the rodent cell kappa locus, for example within the locus or upstream or downstream of the rodent kappa constant region. Preferably the insertion is upstream of the IGL kappa locus constant region, such that the IGL lambda V and J region genes are expressed with the IGL kappa constant region. Suitably the insertion locates the companion animal genes at approximately the same position as the native rodent kappa genes, potentially deleting or displacing them, for example there being the same or substantially the same distance from the last inserted 3' lambda J gene to the rodent kappa constant region gene as there is from the last rodent kappa 3' J gene to the kappa constant region. In one aspect the insertion is within 1, 2, 3, 4, 5, 6, 7, 8, 9, or 10kb of the boundary (upstream or downstream) of the rodent immunoglobulin kappa locus. The mouse kappa light chain is naturally expressed at a higher level than the mouse lambda light chain, and insertion of the companion animal DNA at this kappa locus can provide high levels of expression of the companion lambda chain V region genes.

In one aspect the rodent genome is homozygous for the insertion of companion animal genes at one, or both, or all three immunoglobulin loci.

In another aspect the rodent genome may be heterozygous for the insertion of companion animal genes at one, or 2, or three immunoglobulin loci.

In particular the rodent genome may be heterozygous for the insertion of companion animal genes at the kappa locus.

In one aspect the inserted DNA is capable of being expressed with different rodent constant regions through isotype switching.

In one aspect the inserted companion animal DNA is capable of being expressed with a different rodent constant region through trans-switching.

In one aspect the companion animal is a dog, and the rodent genome comprises canine kappa variable region genes, wherein all of the canine kappa variable region genes in the rodent genome are located upstream of constant region with which the variable region gene is expressed, such as upstream of the rodent kappa constant region or such as upstream of a canine kappa constant region.

In another preferred aspect the companion animal is a horse, and the rodent genome comprises equine kappa variable region genes, wherein all of the equine kappa variable region genes in the rodent genome are located upstream of the constant region with which the variable region gene is expressed, such as upstream of the rodent kappa constant region or such as upstream of an equine kappa constant region.

In one aspect the companion animal DNA is inserted at the rodent wild-type constant region located at the wild type locus, suitably between the rodent constant region and the host VDJ or VJ region. In one aspect the IGH variable region genes are inserted downstream of the heavy chain J region, and upstream of the Emu enhancer.

In one aspect the rodent is a mouse and IGH variable region genes are inserted downstream of the mouse heavy chain J region, and upstream of the Emu enhancer. In one aspect the insertion of IGH V region genes is made at position 114666435 of the mouse genome, on mouse chromosome 12. In one aspect the insertion of IGL lambda V region genes is made at position 19047551 of the mouse genome, on chromosome 16. In one aspect the insertion of IGL kappa V region gene or genes is made at position 70674755 of the mouse genome, on chromosome 6.

In one aspect the rodent is a mouse and the genome comprises at least canine V4-1, V3-2, V3-3 and V3-4 IGH variable region genes.

In one aspect the rodent is a mouse and the genome comprises at least canine V4-1, V7-2, V3-3, V2-4, V2-5, V2-6, V2-7, V2-8, V2-9, V2-10 and V2-11 IGL kappa variable region genes.

In one aspect one the rodent or rodent cell is a mouse or mouse cell and one or more or all of the canine kappa V genes 4-S17, 2-S16, 3-S15, 2-S14 , 2-S13 and 2-S12 are located upstream of the kappa constant region of the rodent.

In one aspect the rodent is a mouse and the genome comprises at least canine V3-1, V3-2, V3-3, V3-4, V4-5 and V4-6 IGL lambda variable region genes.

In one aspect the rodent is a mouse and the genome comprises a deletion of one or some or all of the mouse IGH V region genes, preferably from V1-85 to V5-2.

In one aspect the rodent is a mouse and the genome comprises a deletion of one or some or all of the mouse IGL kappa V region genes, preferably from V3-1 to V2-137.

In one aspect the rodent is a mouse and the mouse heavy chain D and J region genes are retained in the genome upstream of the inserted companion animal heavy chain variable region genes.

In one aspect the rodent genome is modified to reduce or prevent expression of fully rodent antibodies that have both variable and constant regions from the rodent. This may be by inversion of all or part of the rodent VDJ region, or by a deletion of, or an insertion into, the endogenous rodent VDJ or VJ region of the genome. In one aspect the rodent VDJ or VJ region or a part thereof is deleted. In one aspect all or a number of the rodent V region genes are deleted, such as at least 50%, preferably at least 75% or at least 90%, or all of the rodent IGH genes and/or rodent IGL kappa VJ region genes and/or rodent lambda VJ genes. In one aspect the rodent IGL lambda genes are not deleted from the rodent genome.

In one aspect one or both alleles of the rodent kappa locus are deleted or inactivated, in whole or in part, by insertion of companion animal DNA at the rodent kappa locus.

In one aspect the rodent kappa locus is inactivated wholly or partially, for example, by insertion, or by deletion or by inversion.

In one aspect the rodent lambda locus is inactivated, wholly or partially, for example, by insertion, or by deletion or by inversion.

In one aspect the rodent heavy chain locus is inactivated, wholly or partially, for example, by insertion, or by deletion or by inversion.

The companion animal variable region gene(s) are suitably inserted upstream of rodent constant region, the latter comprising all of the DNA required to encode the full constant region or a sufficient portion of the constant region to allow the formation of an effective chimaeric antibody capable of specifically recognising an antigen. Reference to a chimaeric antibody or antibody chain having a rodent constant region herein therefore is not limited an antibody chain having the complete constant region, or a complete constant region locus, but also includes chimaeric antibodies or chains which have a part of the constant region, or constant region locus, sufficient to provide one or more effector functions seen in antibodies occurring naturally in the rodent. Effector functions include the ability to interact with Fc receptors, and/or bind to complement. This teaching also applies to rodents and cells and methods of the invention in which variable region DNA is located in the host genome such that it forms a chimaeric antibody chain with all or part of a rodent constant region to form an antibody chain or a part thereof.

Preferably the rodent genome comprises all of the companion animal lambda constant region DNA and intervening regions.

The rodent constant region expressed with the companion animal variable region is preferably the rodent wild-type constant region located at the wild type locus, as appropriate for the companion animal heavy or light chain VDJ or VJ.

In one aspect at least one rodent enhancer or other control sequence, such as a switch region, is maintained in functional arrangement with the rodent constant region. In this way the effect of the enhancer or other control sequence may be exerted in whole or in part in the cell or transgenic rodent.

In one aspect one or more rodent control sequences, such as the Emu enhancer sequence, is maintained upstream of the rodent Mu constant region, suitably in its native position with respect to the distance from the constant region.

In one aspect one or more rodent control sequences such as an enhancer sequence(s) is/are maintained downstream of the rodent constant region, suitably in its native position with respect to the distance from the constant region.

In one aspect the rodent Smu switch sequence is maintained upstream of the rodent Mu constant region, suitably in its native position with respect to distance from the constant region.

In such location the rodent enhancer or switch sequence is suitably operative *in vivo* with the host constant region sequence(s).

In a further aspect one or more of the promoter elements, or other control elements, of the companion animal V, D or J region genes is/are optimised in the genome to interact with the transcriptional machinery of the rodent.

In one aspect the rodent or rodent cell genome comprises one or more companion animal promoters, or enhancers, and/or other control elements associated with the companion animal V, D or J regions. In one aspect the one or more companion animal control regions, such as promoters or enhancers or switch regions, replace one or more rodent promoters or enhancers or switch regions respectively. The companion animal control sequences are suitably maintained in functional arrangement with a constant region such that the effect of the control sequence(s) may be exerted in whole or in part in the cell or transgenic rodent.

In one aspect at least one or more of the inserted companion animal V, D or J gene segments is associated with a regulatory sequence, such as a recombination signal sequence (RSS), from the same companion animal, optionally wherein the regulatory sequences direct the successful recombination of the V, D or J gene segment or segments.

In this context, the 'same' companion animal is not limited to the precise companion animal from which the companion animal V, D or J gene segments are taken. In one aspect, 'same' companion animal refers to the same breed or species as the companion animal from which the companion animal V, D or J gene segments are taken. In one aspect, it is precisely the same companion animal.

In one aspect, at least one or more of the inserted companion animal V, D or J gene segments is directly associated cis or trans with a regulatory sequence, or flanked on one or both sides with a regulatory sequence, optionally wherein the one or more gene segments is directly flanked by the regulatory sequence.

In one aspect the regulatory sequences comprise a promoter preceding an individual V gene segment, and/or a splice site within an individual V gene segment, and/or a recombination signal sequences for V(D)J recombination downstream of a V gene segment, flanking a D gene segment or upstream of a J gene segment.

In one aspect a V, D or J sequence of the inserted companion animal is flanked by an RSS sequence from the same companion animal. For example, a canine RSS sequence may be used with canine V, D and/or J sequences. It will be appreciated that this can be provided by insertion of a genomic fragment from the companion animal into the rodent genome. In a further aspect, the invention provides a method of replacing, in whole or part, in a rodent cell an endogenous immunoglobulin variable region gene locus with a companion animal gene locus comprising: obtaining a cloned genomic fragment or a synthetic sequence containing, in whole or in part, the companion gene locus comprising at least one V, or D (for the heavy chain) or J gene segments, and at least one associated regulatory sequence, and insertion of the companion animal DNA into the genome of a rodent, suitably at an endogenous mouse immunoglobulin locus, preferably the heavy or light chain rodent locus corresponding to the nature of the inserted companion animal DNA.

In one aspect, the inserted companion animal DNA comprises at least 5kb of genomic DNA, at least 10kb, at least 15kb, 20kb or more from the companion animal.

In one aspect the rodent cell of the invention is a rodent ES cell, rodent hematopoietic stem cell or other cell capable of developing into a rodent able to produce a repertoire of antibody chains comprising a variable region encoded by companion animal DNA, such as chimaeric antibody heavy chains or chimaeric antibody light chains, or fully companion animal antibody chains or antibodies encoded by companion animal variable regions having variable and constant regions.

In one aspect the cell of the invention is a rodent ES cell or an induced pluripotent stem cell (iPS cell).

In one aspect the cell is an isolated rodent cell.

In one aspect the cell is an isolated rodent B cell.

Preferably the rodent cell is a rodent ES cell or iPS cell. Such cells are suitable for the insertion of companion animal DNA to generate rodents expressing antibody chains as described herein.

The ES cell may be of mouse cell strain 129 or C57BL, such as strain C57BL/6N, C57BL/6J, 129S5 or 129Sv strains, or in a cell which has a hybrid genome which comprises 129 or C57BL genomic DNA.

The invention also relates to a cell line which is grown from or otherwise derived from cells as described herein, including an immortalised cell line.

The cell or cell line of the invention may comprise companion animal V, (D) or J genes in germline configuration or after rearrangement following *in vivo* maturation.

The invention also relates to a cell or cell line expressing an antibody chain which is obtainable by immunising a rodent of the invention with an antigen, such as a chimaeric antibody heavy chain.

The invention also relates to a cell or cell line that express an antibody chain having a companion animal variable region, preferably associated with a companion animal constant region, wherein the nucleic acid sequence of the variable region of that antibody may be identified, or has been identified, by immunising a rodent of the invention with an antigen and obtaining an antibody chain or the sequence of the antibody chain from the rodent or a rodent cell. The expressed antibody chain is preferably a fully canine antibody chain, or a fully equine antibody chain, or a fully feline antibody chain, in which the variable region from the companion animal is expressed in a rodent of the invention associated with a constant region from the same companion animal (rather than a rodent constant region). The cell or cell line expressing the antibody chain or antibody may be a CHO cell, or other mammalian cell line suitable for the production of a therapeutic for human or animal use.

The cell may be immortalised by fusion to a tumour cell to provide an antibody producing cell and cell line, or be made by direct cellular immortalisation.

The present invention also relates to vectors for use in the invention. In one aspect such vectors are bacterial artificial chromosomes (BACs) comprising all or a part of the companion animal IG locus. It will be appreciated that other cloning vectors may be used in the invention, and therefore reference to BACs herein may be taken to refer generally to any suitable vector. The vector may comprise one or more selectable markers and/or one or more site specific recombination sites. In one aspect the vector comprises 2 or more, such as 3, heterospecific and incompatible site specific recombination sites. In one aspect the site specific recombination sites may be loxP sites, or variants thereof, or FRT sites or variants thereof. In one aspect the vector comprises one or more transposon ITR (inverted terminal repeat) sequences.

Suitable BACs containing canine DNA are available as the CHORI-82 BAC library from the BACPAC Resources Center of the Children's Hospital Oakland Research institute

Suitable BACs containing feline DNA are available as the FCAB library from Amplicon Express

Suitable BACs containing equine DNA are available as the CHORI-241 BAC library from the BACPAC Resources Center of the Children's Hospital Oakland Research institute ...

The invention relates to a method for producing a rodent or rodent cell, the method comprising inserting into a rodent cell genome one or more companion animal IGH V region genes, one or more companion animal IGH D region genes and one or more companion animal IGH J region genes, wherein the rodent or rodent cell is capable of expressing the companion animal variable region gene(s) in combination with a constant region to form an antibody chain.

The invention also relates to a method for producing a rodent or rodent cell, the method comprising inserting into a rodent cell genome one or more companion animal IGL V region genes, and one or more companion animal IGL region J genes, wherein the rodent or rodent cell is capable of expressing the companion animal variable region gene(s) in combination with a constant region to form an antibody chain.

Preferably the method relates to inserting both light chain and heavy chain companion animal VDJ and VJ region genes respectively such that an antibody is produced in which both light and heavy chains have a variable region derived from expression of companion animal DNA.

The invention also comprises a method for producing a rodent or rodent cell, the method comprising inserting multiple companion animal DNA fragments sequentially into a rodent cell genome, wherein the inserted fragments form a contiguous insertion in the rodent cell, i.e. they are joined together directly without intervening sequences.

In one aspect the insertion process commences at a site where an initiation cassette has been inserted into the genome of a cell, such as an ES cell. In one aspect the initiation cassette is inserted in the rodent heavy chain locus, for use in insertion of human heavy chain DNA. Similarly an initiation cassette may be inserted in the rodent light chain locus, for use in insertion of human light chain VJ DNA. The initiation cassette may be located between the last J and the C region of the rodent heavy and kappa chains. The initiation cassette may be located downstream of, and on the same chromosome as, the rodent kappa IGL locus, for use in insertion of IGL lambda companion animal genes.

The initiation cassette suitably comprises a unique contiguous locus in the rodent genome into which the insertion of companion animal DNA can be made.

In one aspect the insertion of a first DNA fragment into an initiation cassette may be followed by insertion of a second DNA fragment into a portion of the first DNA fragment. Subsequent insertions may be made into at least a part of the previously inserted DNA fragment.

In one aspect the method comprises targeted insertion of an initiation cassette into a rodent genome by homologous recombination, insertion of a first DNA sequence into a least a portion of the initiation cassette by site specific recombination, insertion of a second DNA sequence into at least a portion of the first DNA sequence and optionally insertion of a further one or more DNA sequences into a least a portion of the preceding DNA sequence, to build up a contiguous DNA fragment in the target comprising companion animal DNA. The insertion of a DNA fragment into into at least a portion of the earlier fragment may be by site specific recombination, for example by recombinase mediated cassette exchange (RMCE). The method may comprise both homologous recombination (e.g. for the initial step of insertion of an initiation cassette) and site specific recombination, such as RMCE (e.g. for one or more subsequent insertion events). Site specific recombinase systems are well known in the art and may include Cre-lox, and FLP/FRT or combinations thereof.

In one aspect the inserted companion animal DNA is built up in the genome of a cell, such as an ES cell, in a stepwise manner using 2, 3, 4, 5, 6, 7, 8, 9, 10, 11, 12, 13, 14, 15, 20 or more separate insertions for each heavy chain or light chain region. Companion animal DNA fragments are suitably inserted at the same or substantially the same cell locus, e.g. ES cell locus, one after another, to form a complete VDJ or VJ region, or a part thereof.

The present invention also relates to cells and rodents comprising intermediates in the process, whose genomes may comprise only a partial companion animal VDJ or VJ region, such as only companion animal variable region gene DNA.

Methods for targeted insertion of exogenous DNA at the endogenous mouse locus are well known in the art, such that inserted V, D and J genes can be expressed with the host constant region. See Murphy et al, Vol 111 no 14, 5153-5158, doi: 10.1073/pnas.1324022111; MacDonald et al vol. 111 no. 14, 5147-5152, doi: 10.1073/pnas.1323896111; and Lee et al , Nature Biotechnology Volume: 32, Pages:356-363 Year published:, 2014 DOI:, doi:10.1038/nbt.2825.

In particular, the method for producing a transgenic rodent comprises the insertion of companion animal VDJ or VJ region genes as disclosed herein upstream, or even downstream, of the corresponding rodent mammal constant region by step-wise insertion of multiple DNA fragments by sequential recombinase mediated cassette exchange (SRMCE).

In one aspect a correct insertion event is confirmed before moving to the next step of any multistep cloning process.

In one aspect the companion animal is a dog, the rodent is a mouse and the canine IGL kappa V region genes naturally located downstream of the canine kappa constant region are inserted upstream of the rodent kappa constant region, preferably upstream of and in the same orientation as the canine IGL kappa V genes found naturally upstream of the canine IGL kappa constant region.

In one aspect the rodent is able to generate a diversity of at least 1 X 10⁶ different functional chimaeric immunoglobulin sequence combinations.

In one aspect rodents, ES cells carrying one or more chimeric loci are used to make chimaeras in which the host embryos are generated from a RAG-1-deficient background, or other suitable genetic background which prevents the production of mature host B and T lymphocytes. This enables all the B and T cells to be derived from the injected ES cells.

In one aspect the preferred rodent is a mouse, and cells of the invention are mouse cells or ES cells. In another aspect the preferred rodent is a rat, and cells of the invention are rat cells or ES cells.

The ES cells of the present invention can be used to generate animals using techniques well known in the art, which comprise injection of the ES cell into a blastocyst followed by implantation of chimaeric blastocysts into females to produce offspring, which can be bred to produce heterozygous offspring which are then interbred to produce homozygous recombinants having the required insertion. In one aspect the host blastocysts are Rag-deficient.

The invention relates to a chimaeric rodent generated by injection of an ES cell of the invention into a blastocyst followed by implantation of chimaeric blastocystys into a rodent female to produce offspring.

In one aspect the rodent or rodent cell is a mouse or mouse cell and the mouse ADAM6a and ADAM6b genes are present in the mouse genome, and have not been previously deleted then reinserted from the IGH locus.

In one aspect the rodent ADAM6a and ADAM6b genes are located at a position 5' of the inserted one or more companion animal V, D and J genes.

In one aspect the rodent IGH D and J genes are present in the rodent genome. In one aspect the rodent IGH D and J genes have not been deleted from the rodent genome. In one aspect the rodent IGH D and J genes are located at a position 5' of the inserted one or more companion animal V, D and J genes.

In one aspect the canine alleles used in the present invention are the reference alleles for each canine gene. These are those of CanFam3.1 - see assembly accession - GCA_000002285.2, produced September 2011 and last updated May 2016.

Surprisingly, we have determined from genome data from 107 dogs across 19 breeds that there is minimal breed variation, and that the reference allele (from a Boxer dog) is found 76% of the time across the sample, and where non-reference alleles are typically found as heterozygotes with the reference allele. This means that an antibody population generated from a rodent comprising canine reference alleles will be widely applicable for use across different canine breeds.

Therefore the invention relates to a rodent cell or rodent as disclosed herein in which at least 90%, at least 95% and preferably all of the companion gene segments that have been inserted are the canine reference alleles from CanFam 3.1.

Preferably the rodent or rodent cell includes an allele which is not the reference allele (not the "*01 allele") of one or more of the following gene segments: IGKV2-S13, IGLV1-57, IGLV1-68, IGLV1-72, IGLV1-88, IGLV1-96, IGLV8-60, IGLV8-90, IGLV8-120. Preferably the rodent or rodent cell genome comprises 2, 3, 4, 5, 6, 7, 8 or all 9 of these non-reference alleles.

In one aspect, the rodent or rodent cell comprises one or more reference alleles of the companion animal gene segments, such as 2, 3, 4, 5, 6, 7, 8 or 9 reference alleles.

The invention also relates to an antibody chain or part thereof, obtained or obtainable as disclosed herein, having a variable region which is derived from the expression of a canine reference allele, in the treatment of a disease, in particular in a canine breed other than that of the breed of the reference genome. In one aspect, at least 50, 60, 70, 80, 90 or 100% of the inserted V gene segments are canine V gene reference alleles, and/or at least 50, 60, 70, 80, 90 or 100% of the inserted D gene segments are canine D gene reference alleles, and/or at least 50, 60, 70, 80, 90 or 100% of the inserted J gene segments are canine J gene reference alleles, and combinations of these. Preferably, at least 90%, such as 100%, of the inserted canine gene segment alleles are the reference allele for that gene segment.

The invention also relates to a rodent having inserted canine V, D and J segments as disclosed herein in the generation of an antibody or antibody chain, or part thereof, for use in the prevention or treatment of disease in a different canine breed, and to the use of a rodent having inserted canine V, D and J segments as described herein in the generation of an antibody chain, or part thereof, for use in the prevention or treatment of disease in a different canine breed.

The invention also relates to a rodent having one or more or all canine V, D and J segments, as described herein, from a boxer dog breed. The invention further provides the use of an antibody or fragment thereof obtained or obtainable from any rodent having genomic canine gene segments, as described herein, the antibody being expressed at least in part from canine DNA, in the prevention or treatment of disease in a canine breed which is different from the breed used in the canine gene segments.

For example, where the inserted canine DNA is from the boxer, the use is in a canine species other than boxer.

The invention also relates to:
(i) a rodent having canine V, D and J segments, as described herein, from a boxer dog breed; preferably with one or more boxer gene segments which are reference alleles;
(ii) a rodent having canine V, D and J segments, as described herein, from a dog breed other than a boxer, preferably with one or more V, D and or J gene segments which are the same as that of a boxer dog (i.e. the dog has the reference allele);
(iii) a cell, such as a B cell, hybridoma, CHO or other suitable cell, expressing an antibody or antibody chain from such a rodent in (i) or (ii), wherein the antibody comprises at least some amino acids that are expressed from canine V, D and J DNA (or canine V and J DNA for the light chain); preferably expressed from a reference allele;
(iv) a fully canine antibody or antibody chain comprising the antibody variable region of the antibody above in (iii), which may be generated for example by expressing a DNA encoding the canine variable region with DNA encoding a canine constant region;
(v) an expression cell, such as a CHO cell, comprising the DNA encoding all or a part of such a fully canine antibody,

Preferably the boxer V, D and J gene segments are from the CHORI-82 BAC library.

In another aspect the invention provides for an antibody or antibody chain, and use of an antibody, or antibody chain, having a canine variable region, in the prevention or treatment of a disease in a different canine breed, wherein the antibody or chain is obtained or obtainable from a rodent as disclosed herein, wherein the V gene segment used to generate the canine variable region is a canine reference allele, and preferably wherein the antibody or antibody chain is effective in at least 50%, such as 60% or at least 70% of different canine breeds. Preferably the canine V gene segment from heavy and/or light chain is from a boxer. Preferably the D and J gene segments are also boxer gene segments.

### The invention relates to:

A method for producing antibody chain specific to a desired antigen, the method comprising immunizing a rodent as disclosed herein with the desired antigen and recovering the antibody chain, singularly or as part of a complete antibody, or recovering a cell producing the antibody chain singularly or as part of a complete antibody (see e.g. Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259). Suitably an immunogenic amount of the antigen is delivered. The invention also relates to a method for detecting a target antigen comprising detecting an antibody produced as above with a secondary detection agent which recognises a portion of that antibody.

A method for producing an antibody chain or antibody specific to a desired antigen and derived from a single species of companion animal, the method comprising immunizing a rodent comprising companion animal genes as disclosed herein and then replacing the rodent constant region of the antibody chain with a companion animal constant region from the same companion animal, suitably by engineering of the nucleic acid encoding the antibody. This can be done by standard cloning techniques at the DNA level to replace the non-human mammal constant region with an appropriate human constant region DNA sequence - see e.g. Sambrook, J and Russell, D. (2001, 3'd edition) Molecular Cloning: A Laboratory Manual (Cold Spring Harbor Lab. Press, Plainview, NY) or by direct nucleic acid synthesis.

A method for producing an antibody chain or part thereof, the antibody chain having a companion animal variable region, the method comprising expressing in a cell a DNA encoding the antibody chain, or a part thereof,
wherein the sequence of the DNA encoding the variable region of the antibody chain is obtained from, or is obtainable by, immunising a rodent of the invention with an antigen so that antibody chains are produced,
optionally including the subsequent steps of:
   purifying and/or isolating the antigen receptor chain, and
   optionally then formulating the antigen receptor chain into a pharmaceutically acceptable formulation suitable for administration into a companion animal.

An antibody or antibody chain, or part thereof, or a nucleic acid encoding an antibody chain or a part thereof, which has been obtained or is obtainable from a rodent or cell according to the present invention.

The invention also relates to a part of, or whole, immunoglobulin molecule comprising canine variable domains and rodent constant domains derived from a B cell from a rodent as disclosed herein, as is a hybridoma cell obtainable or obtained from that B cell, and a part of, or whole, immunoglobulin molecule comprising canine variable domains and rodent constant domains obtainable from or obtained from that hybridoma cell. The invention also relates to identifying the canine variable regions by single cell sequencing and make a synthetic vector to express a full antibody chain with the corresponding canine constant region. The invention also relates to obtaining these sequences by PCR and joining them to an appropriate constant region by a suitable molecular biology technique such as but not limited to bridge-PCR and Gibson cloning. An antibody or antibody or chain, or part thereof, obtained or obtainable according to the invention, for use in treatment of a companion animal.

A method of treatment of a companion animal comprising delivery of a suitable antibody or antibody chain or part thereof, obtained or obtainable according to the invention, to a companion animal in need thereof. In particular the invention relates to a method of medical treatment comprising delivering to a companion animal in need thereof an antibody chain or antibody, or part thereof, where at least the variable region of the antibody is has been obtained or otherwise identified by immunising a rodent of the invention comprising companion DNA V (D) J region genes with an antigen.

In one aspect the invention relates to chimaeric companion animal antibodies and antibody chains, having a rodent constant region and a companion animal variable region, as well as fragments and functional derivatives of said antibodies and chains, and use of said antibodies, chains and fragments in medicine, including diagnosis, and in vitro or ex vivo studies. Functional antibody fragments can include a fragment that is capable of specific binding to an antigen. A functional antibody fragment may be, for example, a FAB or a single chain variable fragment (scFv). In a further aspect the invention relates to "fully" companion animal antibodies and antibody chains (where "fully" reflects the fact that both the variable and constant regions of the antibody are expressed from companion animal genes of the same species), as well as fragments and functional derivatives of said antibodies and chains, and use of said antibodies, chains and fragments in medicine, including diagnosis, and in vitro or ex vivo studies.

Methods for the generation of both monoclonal and polyclonal antibodies are well known in the art, and the present invention relates to both polyclonal and monoclonal antibodies of chimaeric or fully companion animal antibodies produced in response to antigen challenge in rodents of the present invention.

In a further aspect the invention relates to use of a rodent as described herein as a model for the testing of drugs and vaccines. The invention therefore relates to a method for identification or validation of a drug or vaccine, the method comprising delivering the vaccine or drug to a mammal of the invention and monitoring one or more of: the immune response, the safety profile; the effect on disease.

In a yet further aspect, chimaeric antibodies or antibody chains generated in the present invention may be manipulated, suitably at the DNA level, to generate molecules with antibody-like properties or structure, such as a companion animal variable region from a heavy or light chain absent a constant region, for example a domain antibody; or a companion animal variable region with any constant region from either heavy or light chain from the same or different species; or a companion animal variable region with a non-naturally occurring constant region; or a companion animal variable region together with any other fusion partner. The invention relates to all such chimaeric antibody derivatives derived from chimaeric antibodies identified according to the present invention.

The invention also relates to a kit comprising an antibody or antibody derivative as disclosed herein and either instructions for use of such antibody or a suitable laboratory reagent, such as a buffer, antibody detection reagent.

The invention also relates to a method for making an antibody, or part thereof, the method comprising providing:
(i) a nucleic acid encoding an antibody, or a part thereof, obtained according to the present invention; or
(ii) sequence information from which a nucleic acid encoding an antibody obtained according to the present invention, or part thereof, can be expressed to allow an antibody to be produced.

The present invention also relates to a chimaeric antibody comprising a companion animal variable region and a rodent constant region (optionally a C gamma or C mu), wherein the antibody is encoded by a nucleotide sequence corresponding to the nucleotide sequence of a chimaeric heavy chain locus of a cell (optionally a B-cell, ES cell or hybridoma), the locus comprising a rodent constant region nucleotide sequence and a rearranged VDJ nucleotide sequence produced by the in vivo rearrangement of a companion animal V region, a companion animal D region and a companion animal J region, the companion animal V region being selected from canine IGH V4-1, V3-2, V3-3, V3-4, V3-5 IGH variable region genes.

Optionally, the J region is any of canine JH1, JH2, JH3, JH4, JH5 or JH6.

Optionally the D region is any one of canine DH1, DH2, DH3, DH4, DH5 and DH6.

In one aspect the antibody comprises any combination exemplified in the Examples and Figures herein. Optionally, the in vivo rearrangement is in a cell (e.g., a B cell or ES cell) from the same rodent species as the constant region sequence (e.g., a mouse B cell or ES cell). The invention also relates to a non-human vertebrate or mammal cell (e.g., a B cell or ES cell or hybridoma) whose genome comprises a chimaeric heavy chain locus as described above in this paragraph.

The invention also relates to a non-human vertebrate or mammal (e.g., a mouse or rat) whose genome comprises a chimaeric heavy chain locus as described above in this paragraph

Antibodies of the invention may be isolated, in one aspect being isolated from the cell or organism in which they are expressed.

The invention also relates to parts of antibody chains. In particular the part comprises at least the variable region of the antibody. This may be expressed from a cell, in particular for antibody production. The part may comprise the Fab region of the antibody, or may comprise at least the CDR regions.

The invention relates to a method for generation of an antibody or antibody chain, the method comprising immunizing a rodent as described herein with an antigen obtainable from a companion animal which is the same as the source of the companion animal DNA present in the rodent genome. For example, a canine antigen can be used to immunise a rodent comprising canine V, D and J genes as disclosed herein.

The invention thus also relates to a rodent immunised with an antigen from a companion animal which corresponds to the source of the companion animal DNA present in the rodent genome,

The same exact companion animal does not need to be used. For example a canine antigen taken from one breed can be used to immunise a rodent comprising canine DNA from a different breed. An antigen from the same canine breed may also be used.

In an alternative, the antigen may be from a pathogen such as a bacteria or virus that is known to infect the companion animal. For example, an antigen from a pathogen that infects and causes disease in a dog can be used to immunise a rodent comprising canine V, D and J genes as disclosed herein.

The invention therefore relates to a rodent immunised with an antigen which causes disease in a companion animal which corresponds to the source of the companion animal DNA present in the rodent genome.

In a further aspect the antigen may be a companion animal equivalent of a human antigen which is associated with disease in humans, and preferably which has been validated as a target for prevention or treatment of disease in humans.

In one aspect the invention provides an antibody chain or fragment thereof, obtained or obtainable by immunisation of a rodent with an antigen as described herein.

The invention also relates to nucleic acid, such as DNA or RNA, encoding said antibody, antibody chains, or parts thereof. In particular the part may be the variable portion of the antibody chain, which is that part encoded by the companion animal DNA within the rodent.

The invention also relates to a cell, such as a B cell, or a hybridoma, or an expression cell line (e.g. a CHO cell), expressing a partially or fully canine antibody chain, or a partially or fully canine portion of an antibody chain, the DNA or protein sequence of which is obtainable or has been obtained from a rodent as described herein.

It will be appreciated that once an antibody of interest has been identified from an immunised rodent, it is standard in the art to be able to identify the DNA sequence encoding that antibody from a B cell, and to express the antibody, or part thereof, from that sequence, or indeed from another DNA sequence which can express the same protein as a result of genetic code redundancy. In particular the canine variable region can be expressed with a canine constant region to generate a fully canine antibody chain or antibody.

Thus the invention also provides a method for obtaining a fully canine antibody, the method comprising the steps of
immunising a rodent with an antigen as described herein, the rodent having at least one heavy chain canine immunoglobulin V gene segment, at least one canine heavy chain D gene segment and at least one canine heavy chain J gene segment and/or having at least one light chain canine immunoglobulin V gene segment and at least one canine heavy chain J gene segment;
selecting an antibody chain or chains produced by the rodent having a variable antibody region encoded by canine DNA; and
expressing that variable antibody region from DNA in an expression cell line to express an antibody or antibody chain or part thereof, preferably wherein the antibody or antibody chain or part thereof is fully canine, and for example comprises a canine variable region and a canine constant region;

optionally purifying the antibody, antibody chain or part thereof; and
optionally further formulating the antibody or chain or part thereof with a pharmaceutically acceptable excipient, suitable to allow administration to a companion animal in need thereof.

The invention also relates to the use of such antibodies or antibody chains or fragments thereof in therapy in companion animals.

The above approach applies equally to the other preferred companion animals, such as cats and horses. Feline antigens can be used in the equivalent feline/rodent model and equine antigens in the equivalent equine/rodent model, and all other aspects of the invention may be applied equally to cats and horses.

The invention relates to a method for the generation of a cross-reactive antibody or antibody chain, the method comprising immunizing a rodent comprising companion animal DNA as disclosed herein with an antigen of interest from said rodent and a corresponding antigen from the companion animal, wherein the rodent comprises a gene knockout for the gene encoding said antigen. The antibody population raised from said immunised rodents can comprise antibodies that are capable of binding both the rodent and companion animal antigen, i.e. are cross-reactive.

Thus, in a further aspect the invention relates to a cross-reactive antibody or antibody fragment derived from a rodent comprising companion animal DNA as described herein, immunised with a rodent antigen of interest and the corresponding antigen from the companion animal, wherein the rodent has a gene knockout for the gene encoding said antigen.

In one aspect, the rodent is sequentially or simultaneously immunised with the rodent antigen, either directly with a protein or peptide fragment thereof, or with a vector coding for the relevant antigen or fragment thereof, or an isogenic cell line expressing the desired antigen, and the corresponding antigen from the companion animal.

In one aspect, the rodent is a mouse and the companion animal is a dog, cat or horse.

Cross-reactive antibodies are very important in the field of drug discovery. Cross-reactive antibodies can be quickly validated as drug candidates for a species in which the antibody is reactive by using the other species for which the antibody is reactive as a model, without the need for antibody modification.

The invention also relates to the use of a canine antigen for immunisation of a rodent as claimed herein, and relates to a canine antigen for immunisation of a rodent as claimed herein, wherein the canine antigen has a family gene or protein equivalent in humans, preferably which is therapeutically validated meaning that antibodies against the human equivalent antigen have been shown to be effective in the treatment of disease.

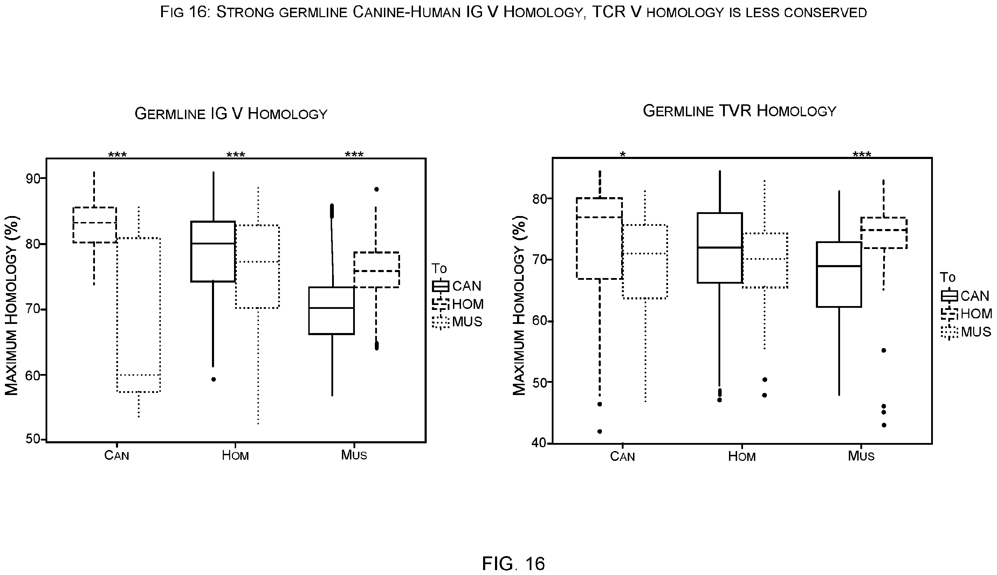

The present invention discloses for the first time the strong homology between the canine and human Ig variable regions. See Figure 16. [Strong germline Canine-Human IG V Homology, TCR V homology is less conserved]. This information suggests a rodent model containing canine variable region DNA may be able to utilise canine regulatory sequences, for example, rather than requiring mouse regulatory sequences, and this is because humanised mice are able to use human regulatory sequences in mice. Indeed, we have now experimentally verified that control sequences and RSS sequences, of inserted canine genomic DNA can be recognised by a rodent and used to express chimaeric antibodies in the rodent.

These findings by the present inventors are surprising and not expected based on the evolutionary divergence of canines and humans.

It is well-known that within the assigned phylogeny, mice and humans are more closely related than either are to (for example) carnivores, including cats and dogs, and horses. This is likely to be the reason why the use of human regulatory sequences in chimeric mice models with human DNA V, D and J gene segment inserts is successful in said models. The same was not predictable for evolutionarily more diverse animals, such as humans and dogs, on this basis. Indeed, previous attempts to use canine gene segments in mice utilised murine regulatory control sequences (e.g. Trianni - US2017306352).

In one aspect the invention relates to a rodent in which the inserted (companion animal) IGHJ4 and IGHJ6 are the predominant JH gene segments found in the mature B cell antibody population. Preferably the rodent comprises canine V, D and J gene segments, and the J4 and J6 are canine J4 and J6 gene segments. In one aspect of the invention the rodent is able to use 1, 2, 3, 4, 5, 6 or more or all of the different inserted IGH D segments and/or 1, 2, 3, 4, 5, 6 or more or all of the inserted IGH J gene segments in the generation of antibody chains within the rodent antibody repertoire.

The presently tested rodent mice with canine inserted heavy chain D1-6 and J1-6 gene segments are able to utilise all of canine IGH J1-6 and D1-6 in antibody formation.

In one aspect of the invention the rodent utilises more IGLJ1 than other light chain J gene segments.

In one aspect of the invention the rodent comprises companion animal DNA from a dog, and the the rodent has one or more of the following characteristics:
the rodent expresses more chimaeric antibody heavy chains expressed from IGHJ4 than individually, any of IGHJ1, 2, 3, 5 or 6;the rodent expresses more chimaeric antibody heavy chains expressed from IGHJ6 than, individually, any of IGHJ1, 2, 3, or 5;
the rodent expresses more chimaeric antibody heavy chains expressed from IGHD5 than, individually, any of IGHD1, 2, 3, 4 or 6;
the rodent expresses more chimaeric antibody heavy chains expressed from IGHD2 than, individually, any of IGHD1, 3, 4 or 6;
the rodent expresses an antibody chain from IGHD2 together with IGHJ4;
the rodent expresses an antibody chain from IGHD5 together with IGHJ4;
the rodent expresses more antibody chains from IGHD5 together with IGHJ4 than are expressed from any other combination of canine IGHD and IGHJ segments;
the rodent expresses more chimaeric antibody light chains expressed from IGLJ1 than any other IGL J gene segment.

In one aspect the rodent comprises inserted canine heavy chain gene segments as follows; J1-6, D1-6, V4-1, V3-2, V3-3 and V3-5, optionally with V3-4 (which is a pseudogene).

In one aspect the rodent comprises inserted canine lambda light chain gene segments as follows; J1-9, together with C1-9 (so the complete canine lambda JC cluster), as well as V3-2, V3-3, V3-4, V4-5, V4-6, and optionally V3-1 and V3-7 (which are both pseudogenes).

The rodent of the invention suitably is able to modify the inserted companion animal V, D and J gene segments by N and or P additions, and/or undergoes somatic hypermutation of the inserted companion animal V, D and J gene segments.

The invention also provides a HCDR3, VH domain, antibody heavy chain or antibody wherein the VH domain of the heavy chain or antibody comprises rodent AID-pattern somatic hypermutations and/or mouse dTd-pattern mutations. This pattern can be provided, for example, wherein VH domain is produced in a rodent comprising rodent AID and/or rodent TdT (e.g., endogenous AID or TdT). Mice are preferred rodents.

In one aspect of the invention the variable region of the chimaeric antibody chain is different from the amino acid sequence that would be predicted from the germline sequence of the V, D and J gene segments used to generate the antibody. As such, there has been some degree of somatic hypermutation, and or N /P addition.

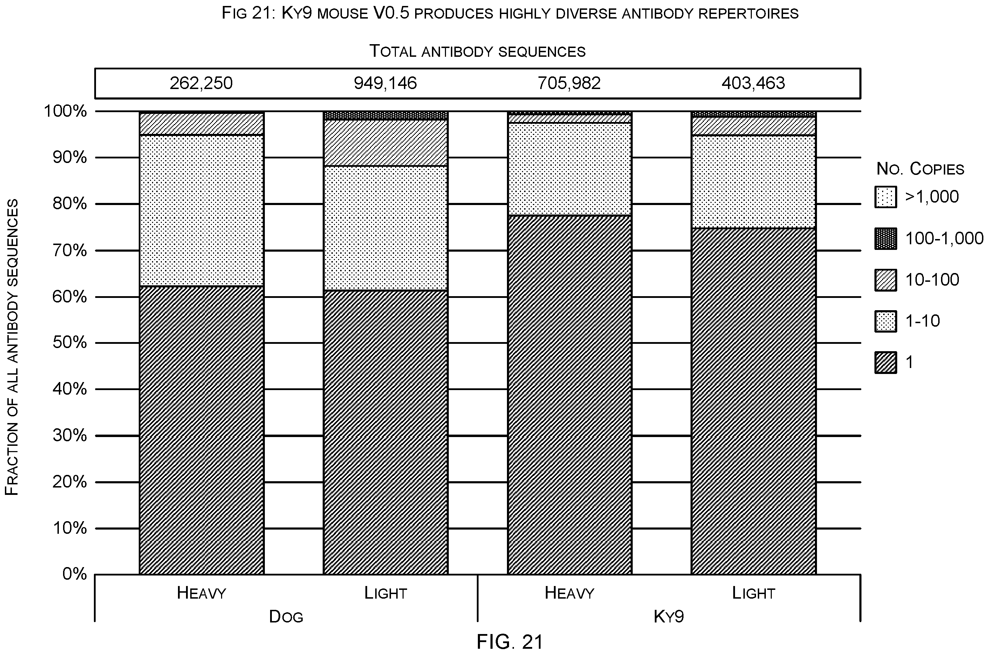

We have surprisingly discovered that, when using the RNA-Sequencing approach of example 4 to interrogate both samples at similar depths, the diversity of the chimaeric antibody population generated using a rodent comprising canine V, D and J gene segments, or V and J segments, as described herein, is greater than the observed antibody diversity of wild type dog from which the gene segments were derived. Figure 21 shows that there are many more antibody nucleotide sequences that were found only once within the sequenced libraries from the rodent containing canine heavy chain V, D and J gene segments, or light chain V and J segments, than are seen in the dog itself.

As such the invention relates to any rodent as described herein which expresses a population of chimaeric antibody chains wherein each antibody results from the expression of at least one companion animal gene segment, and in which that population is more or at least as diverse as the population of antibodies seen in the corresponding companion animal. In other words, the diversity of the antibody population generated in the rodent is greater or at least as great as that seen in the antibody repertoire of the companion animal.

Preferably the diversity of the antibody population generated in a mouse having canine DNA V, D and J gene segments, or light chain V and J segments, is greater or at least as great as that seen in the antibody repertoire of a dog.

Suitably population diversity is assessed by the number of unique antibody sequences that are present in either the heavy chain population or light chain population, or both.

The invention also relates to a rodent comprising a population of chimaeric antibody chains of which at least 65%, or at least 70% are unique, such as a rodent in which between 65 - 80% of the chimaeric antibody sequences are unique, such as a rodent in which 65- 75% are unique with regards to the sequence as determined by 5' RACE, for example in Example 4.

The rodent may be any rodent disclosed herein, for example in one aspect the rodent comprises inserted canine heavy chain gene segments as follows; J1-6, D1-6, V4-1, V3-2, V3-3 and V3-5, optionally with V3-4 (which is a pseudogene). In one aspect the rodent comprises inserted canine lambda light chain gene segments as follows; J1-9, together with C1-9 (so the complete canine lambda JC cluster), as well as V3-2, V3-3, V3-4, V4-5, V4-6, and optionally V3-1 and V3-7 (which are both pseudogenes). In one aspect the rodent comprises both canine heavy and light chain insertions.

The invention also relates to the use of any rodent (e.g. a mouse) comprising companion animal DNA as disclosed herein in the generation of a repertoire of chimaeric antibody chains or antibodies that is more or at least as diverse as is seen in the companion animal itself, and to a rodent, such as a mouse, comprising companion animal DNA as disclosed herein for the generation of a repertoire of chimaeric antibody chains or antibodies that is more or at least as diverse as is seen in the companion animal itself.

### Preferable aspects of the invention are therefore:

A method for generation of an antibody or antibody chain, the method comprising immunizing a rodent according to claims 1-13 with an antigen obtained or obtainable from a companion animal which is the same as the source of the companion animal DNA present in the rodent genome, wherein the antigen may be a protein antigen, a cell expressing the antigen or nucleic acid encoding the antigen.

A method for generation of an antibody or antibody chain, the method comprising immunizing a rodent according to claims 1-13 with an antigen from a pathogen, such as a bacteria or virus, that infects the companion animal species which is the source of the companion animal DNA present in the rodent genome.

A rodent according to claims 1-13 immunised with an antigen from a companion animal which corresponds to the source of the companion animal DNA present in the rodent genome.

A rodent according to claims 1-13 immunised with an antigen which causes disease in a companion animal which corresponds to the source of the companion animal DNA present in the rodent genome.

A rodent according to claims 1-13 immunised with a companion animal antigen equivalent of a human antigen, the human antigen being which is associated with disease in humans.

A rodent according to claims 1-13 wherein the rodent comprises companion animal DNA from a dog, and the rodent has one or more or all of the following characteristics:
the rodent expresses more chimaeric antibody heavy chains expressed from IGHJ4 than individually, any of IGHJ1, 2, 3, 5 or 6;
the rodent expresses more chimaeric antibody heavy chains expressed from IGHJ6 than, individually, any of IGHJ1, 2, 3, or 5;
the rodent expresses more chimaeric antibody heavy chains expressed from IGHD5 than, individually, any of IGHD1, 2, 3, 4 or 6;
the rodent expresses more chimaeric antibody heavy chains expressed from IGHD2 than, individually, any of IGHD1, 3, 4 or 6;
the rodent expresses an antibody chain from IGHD2 together with IGHJ4;
the rodent expresses an antibody chain from IGHD5 together with IGHJ4;
the rodent expresses more antibody chains from IGHD5 together with IGHJ4 than are expressed from any other combination of canine IGHD and IghJ segments;
the rodent expresses more chimaeric antibody light chains expressed from IGLJ1 than any other IGL J gene segment.

A rodent according to claims 1-13 comprising inserted canine heavy chain gene segments as follows; J1-6, D1-6, V4-1, V3-2, V3-3 and V3-5, optionally with V3-4.

A rodent according to claims 1-13 comprising inserted canine lambda light chain gene segments as follows; J1-9, C1-9, V3-2, V3-3, V3-4, V4-5, V4-6, and optionally V3-1 and V3-7.

A rodent according to claims 1-13 capable of modifying the inserted companion animal V, D and J gene segments by N and or P additions, and/or which displays somatic hypermutation.

A rodent according to claims 1-13 which expresses a population of chimaeric antibody chains, wherein each antibody chain results from the expression of at least one companion animal gene segment, and in which that chimaeric antibody population is more diverse than the population of antibodies seen in the corresponding wild type companion animal.

A rodent according to claims 1-13 comprising a population of chimaeric antibody chains of which at least 65%, or at least 70% are unique, optionally wherein the rodent is a dog.

A rodent according to claims 1-13 in which between 65 - 80% of the chimaeric antibody sequences are unique, such as a rodent in which 65 - 75% are unique.

Use of any rodent (e.g. a mouse) comprising companion animal DNA according to claims 1-13 in the generation of a repertoire of chimaeric antibody chains, or antibodies, that is more diverse than is seen in the companion animal itself

A rodent according to claims 1-13, such as a mouse, for the generation of a repertoire of chimaeric antibody chains or antibodies that is more diverse than is seen in the companion animal itself.

A rodent according to claims 1-13 in which one or more of the inserted companion animal V, D or J gene segments is associated with a regulatory sequence from the same companion animal, optionally wherein the regulatory sequences comprise a promoter preceding individual V gene segments, and/or a splice site, and/or a recombination signal sequences for V(D)J recombination.

A rodent according to claims 1-13 wherein a V, D or J sequence of the inserted companion animal is flanked by an RSS sequence from the same companion animal, and the host rodent RSS sequence is not used.

A rodent according to claims 1-13, containing canine DNA from one breed, for use in the generation of an antibody or antibody chain, or part thereof, for use in the treatment or prevention of a disease in a different canine breed,
Use of a rodent according to claims 1-13, containing canine DNA from one breed, in the generation of an antibody chain, or part thereof, for use in the treatment of a different canine breed.

A rodent according to claims 1-13 wherein one or more or all canine V, D and J segments are from a boxer dog.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps
The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, ABAB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

The present invention is described in more detail in the following non limiting Examples..

### Examples

### Example 1 - Annotation of the canine IG loci

Dogs are an excellent model for human disease, for example the treatment of canine lymphoma is often predictive of the human response to that treatment. However, an incomplete picture of their antigen-receptor (AR) gene loci has restricted their use. This work advances the annotation of the canine AR loci, develops methods to interrogate their evolutionary pressures, and looks into breed-specific features of the loci. A bioinformatic approach alongside unbiased RNA-seq was used to complete the annotation of the canine AR genes, and these sequences were used to query 107 whole genome sequences from 19 breeds. A combination of existing and novel methods were used to analyse diversity and mutation rates across these genes. >5,500 novel alleles were identified across the ~550 gene segments (of which 326 were newly annotated) of the AR loci, yielding insight into AR evolution as well as confirming the greater conservation between dog and human than mouse with either. This work brings our understanding of the genetics and expression of ARs in dogs to the same high standard as that of mice and men, making it only the third species to have all AR loci annotated. The large number of genome sequences serves as a reference for future studies, and has allowed statistically powerful conclusions to be drawn on the pressures that have shaped these loci.

### 1. Introduction

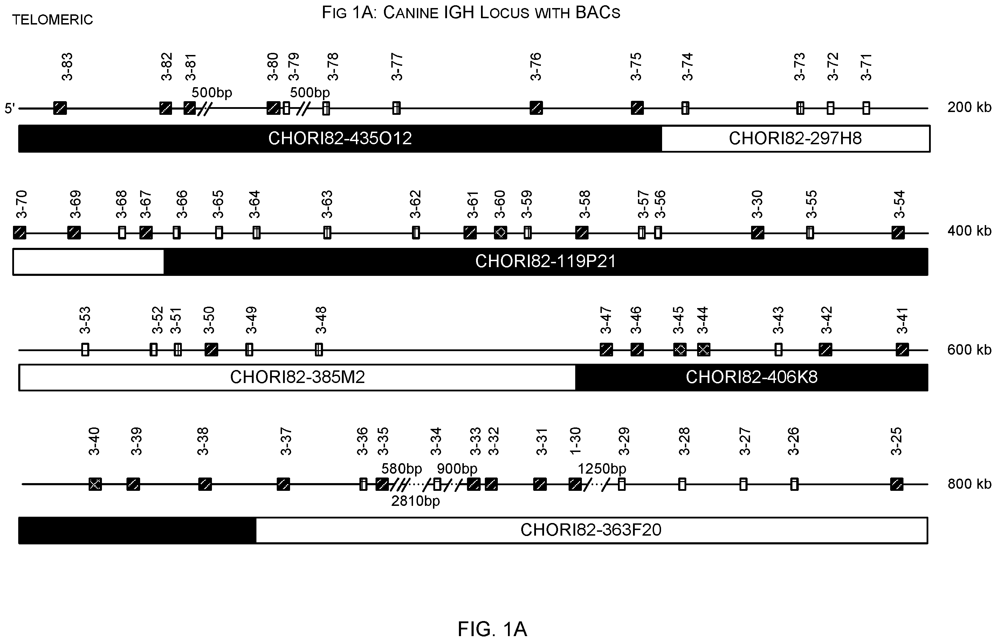

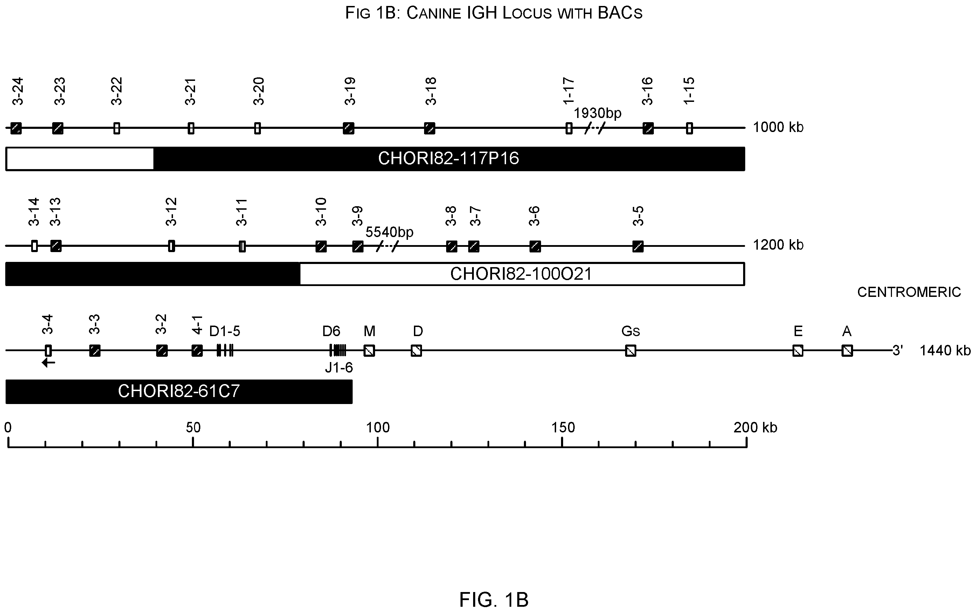

In this study, the canine IGK and IGL were annotated, and the IGH locus was updated **(****Figures 1-****3).** Whole genome sequence data from over one hundred dogs was used to identify five thousand non-reference alleles, and these shed light on the evolutionary pressures that have shaped these loci. Cross-species comparisons have lent further insight and confirmed the dog as a more faithful immune model.

### 2. Materials and Methods

### 2.1.1 Bioinformatic annotation

The loci were initially annotated following a method similar to Das and colleagues, and using equivalent principles to the algorithm of Olivieri and colleagues^{1,2}. Briefly, human and mouse sequences were used to interrogate the canine reference genome (CanFam3.1). Once regions were identified, they were locally searched for AR genes. Initially mouse and human AR gene and RSS consensi were used, but as more canine genes were identified these were used in their stead. The annotation was then verified with and added to based on alignment of the RNA-seq data.

### 2.1.2 Dogs

Peripheral blood samples were secured from twenty six dogs. The samples were the unused clinical excess of veterinarian-mandated blood draws from patients seen at the veterinary hospital of the University of Cambridge. This study received prior approval from the ethics committee of the veterinary school of the University of Cambridge.

### 2.1.3 Sequencing

Mononuclear cells were isolated from the peripheral blood using Ficoll-Paque (GE Healthcare) following the manufacturer's instructions. The cells were processed into mRNA using polyA-pulldown, sheared, and sequenced on a HiSeq 2500 machine (Illumina) using 250bp paired end reads by the core sequencing team at the Wellcome Trust Sanger Institute.

### 2.1.4 Gene Naming

AR genes were divided into families and assigned as functional, pseudogenes, or ORFs using the same criteria as Bao and colleagues³. Family numbers were assigned based on homology to human families, and given a new number in the cases where no obvious match could be found. All gene names were assigned in keeping with the nomenclature system of IMGT⁴.

### 2.1.5 Non-reference alleles

Variant call files mapping to the AR loci from 107 canine whole genome sequences were kindly provided by Steven Friedenberg from the University of Minnesota.

### 2.1.6 Inter- and Intra-species loci alignments

Sequences were masked using RepeatMasker and alignment plots generated using PipMaker ^{5,6}.

### 2.1.7 Phylogenetic analysis

Sequences were aligned using Clustal Omega and the output tree was visualised using the Interactive Tree of Life ^{7,8}.

### 3. Results

### 3.1.1 Gene numbers

Within the previously annotated loci three new IGHJ genes were identified. 162 IGLV genes across seven gene families were identified, with IGLV1 being the largest with 86 members. In keeping with other IGL loci, the J and C genes were found as pairs, nine in total. Nineteen IGKV genes were identified, of which fourteen were IGKV2, alongside five IGKJ and one IGKC gene.

### 3.1.2 Non-reference alleles

Whole genome sequences from 107 dogs from 19 breeds, all aligned to the current reference build (CanFam3.1), were interrogated for novel AR alleles, and 4,074 were identified across the three loci. In terms of allele distribution, the reference allele was found 53,311 times out of the 68,908 alleles called (77%). No significant breed specificity in allele distribution was identified.

The assignment of functional, pseudogene, or ORF was made of all of the novel alleles and compared back to the reference allele. The majority (72.8%) of the new alleles have the same functionality as the reference allele. Taking the functionality of the new allele as the one that is under selection, there were more alleles that were 'loss' (23.8%), i.e. the new allele was a pseudogene, than 'gain' (3.4%), where the new allele is functional. This is unsurprising as there are many more ways to lose functionality than to gain it, and so it is more likely that a mutation will result in that direction of change. Changes to ORF from functional were classed as loss, and changes to ORF from pseudo were classed as gain.

Non-reference V alleles were called 13,129 times across all samples and loci. Interestingly, IGKV2-S13, and eight IGLV genes were only found as non-reference alleles, including in the boxer samples. This is probably an error in the reference genome, but it is also possible that the reference dog happens to carry nine rare alleles that aren't represented in this dataset. Taking the non-reference alleles, if there were no selection pressure then the distribution of change types in the dataset would be the same as their distribution within the alleles themselves. For example, 3.4% of alleles are 'gain', so one would expect gain alleles to be found 446 times in the dataset.

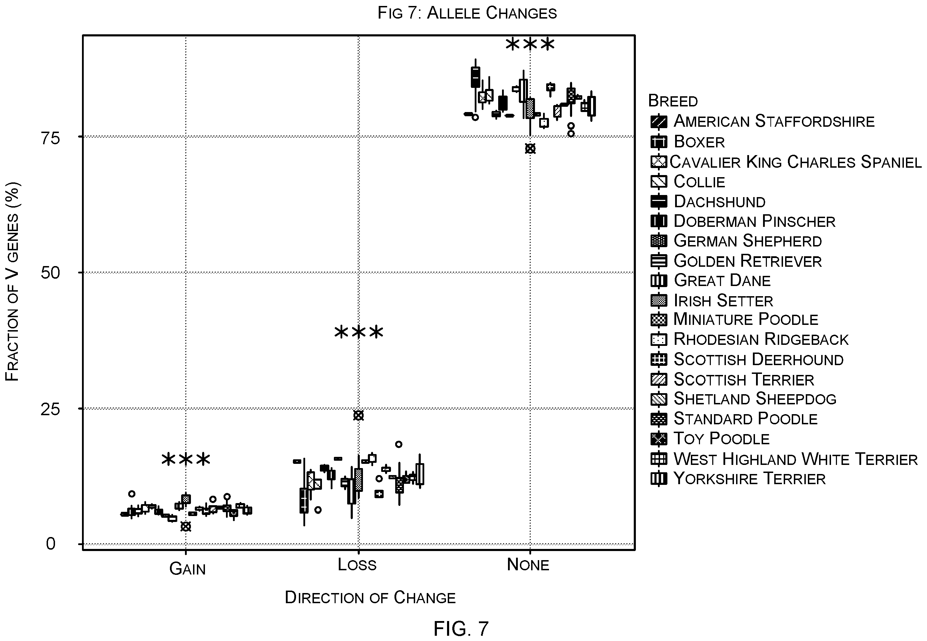

However, loss changes are found less often than expected, and no change and gain are found more often than expected **(****Figure 7****).** Furthermore, the variances were very low across the breeds, lending further weight to the selection pressures on the AR loci having limited breed-dependence. A z test was carried out and the differences between the population and expected means were highly significant, ranging from p = 2.34 x10⁻²⁵ down to p = 0 (the software used to calculate the values is unable to display p values below 1x10⁻²⁵⁰).

### 3.1.3 Locus structure

The canine IGH locus is located just sub-telomeric of chromosome eight, on the antisense strand. This telomeric location is observed in all mammals with the exception of monotremes and marsupials ¹. The light chain loci, however, do not show strong conservation of chromosomal location between human, mouse, and dog.

As with the human and mouse IGH loci, the canine IGH locus has all functional gene segments transcribed in the same sense as the constant regions, with one pseudogene in the reverse transcriptional orientation **(****Figure 1****).** The structure of the IGH locus is similar to that published by Bao and colleagues³. However, three new IGHJ genes have been identified, and there are small differences in the position and expected functionality of the V genes, although absolute ratios and numbers do match. The discrepancy may be due to the use of different builds of the reference genome, with this annotation using the most up to date publically available build (CanFam3.1).

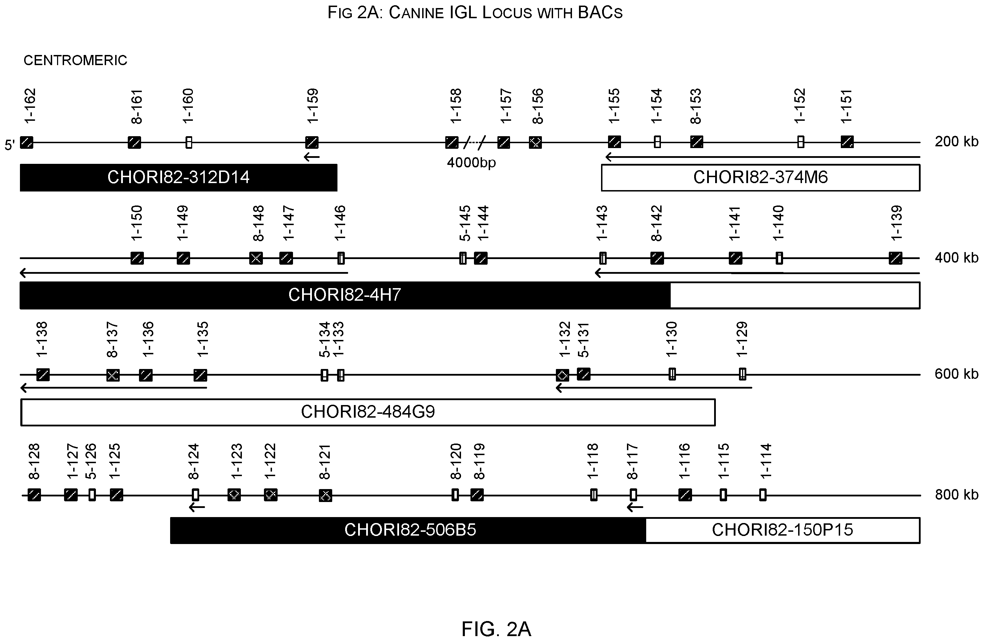

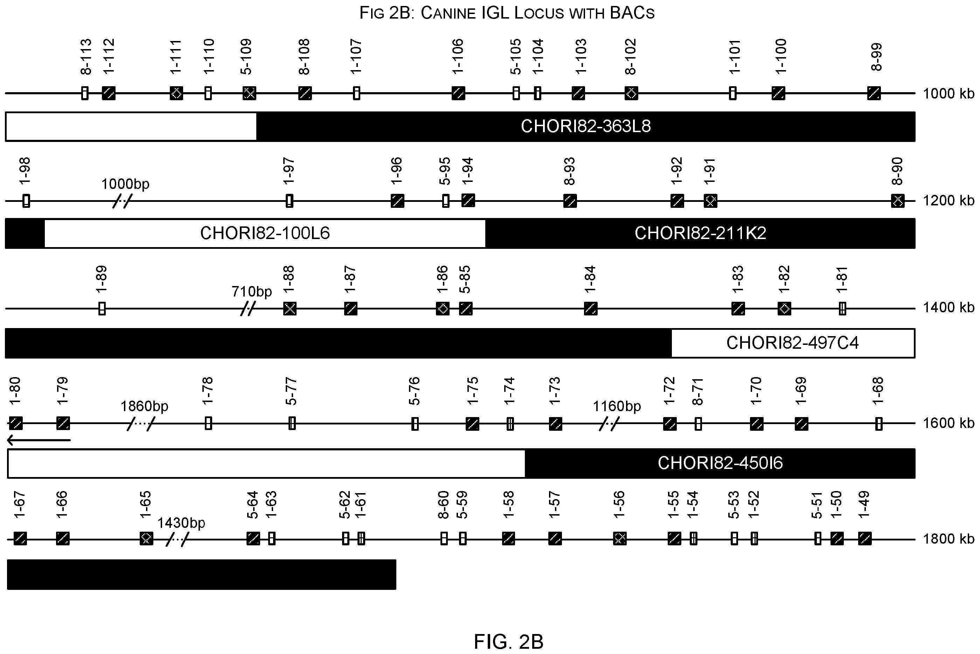

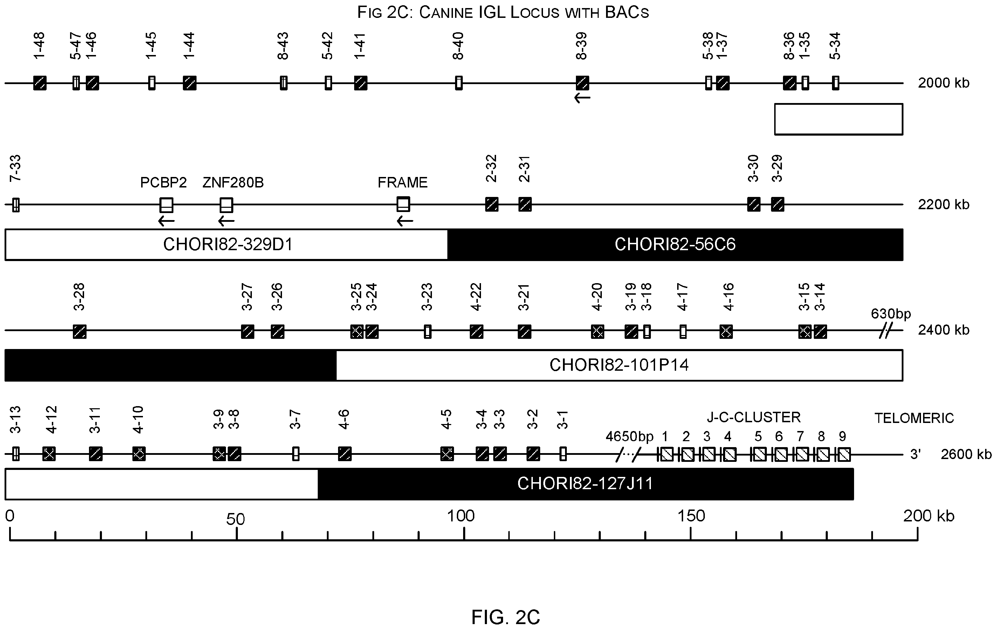

The canine IGK locus is both small (~400kbp) and has an unusual structure **(****Figure 2****).** It has eleven V genes upstream of the J and C genes, where the eight C-distal ones are all functional, from the IGKV2 family, and in the same transcriptional orientation as the J and C genes, unlike the three C-proximal genes. However, it also has eight V genes downstream of, and largely inverted with respect to, the other genes. This is reminiscent of the equine IGL locus, where it was found that orientation had no impact on V gene usage.

Inversions and block duplications appear to be a feature of light chain loci, in particular IGK loci. Not only do the IGK loci of humans, pigs, mice, horses, and dogs, all contain V-genes with the opposite transcriptional orientation to the C gene, but the dog, human, and pig loci have undergone inversional duplication of entire blocks^{10,11}. In the pig and the human loci, the genes in the two blocks have diverged sufficiently little that some or all are known by the same name as their pair in the other block^{10,12}.

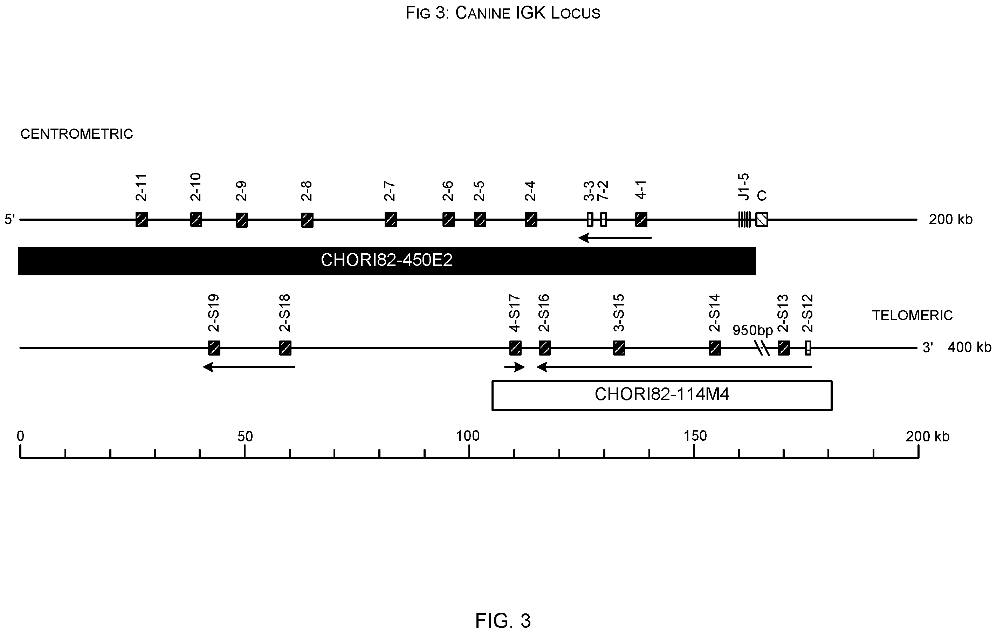

Whilst the human, pig, and mouse IGLV genes maintain their transcriptional orientation along the locus, the canine IGLVs do not¹³. The large (2.6Mbp) canine IGL locus contains many V genes inverted with respect to the J-C cluster **(****Figure 3****).** The inversion appears to be under a degree of location-specific selection pressure, with only three of the 116 most C-proximal IGLV genes in the opposite transcriptional orientation to the J-C genes, but twenty six inverted V genes in the final forty six. This increase is partly down to the block duplications that have occurred, with stretches of IGLV1 and IGLV8 whose members show very high levels of sequence identity in a pattern that is positionally conserved within repeating blocks.

In terms of broader trends, the canine IG loci remain consistent with other published loci. In the IGHVs previously annotated, it was found that the gene to pseudogene ratio was roughly 1:1, which is consistent with the canine IgH locus¹. However, the two light chain loci seem to be closer to 5:1, possibly reflecting different tolerances for pseudogenes between the heavy and light chains. Furthermore, gene number has been found to correlate to biased chain usage in the light chains in most species studied to date^{14,15}. For example the mouse IGL locus only containing nine functional genes, which mirrors its use in only 5% of expressed antibodies ¹⁴. The canine repertoire shows similar bias the other way, with 91% reported usage of the IGK chain, in accordance to the relative sizes of the two light chain repertoires in each species ¹⁵.

### 3.1.4 Inter- and Intra-species loci alignments

Aligning regions of the genome against themselves and against the equivalent regions from other species is an established method of drawing evolutionary insight, and PipMaker is a common tool used for this purpose^{16, 17, 6, 18}. Alignments were carried out using the canine IGK, IGL, and TRA/D loci against themselves and against their respective mouse and human loci.

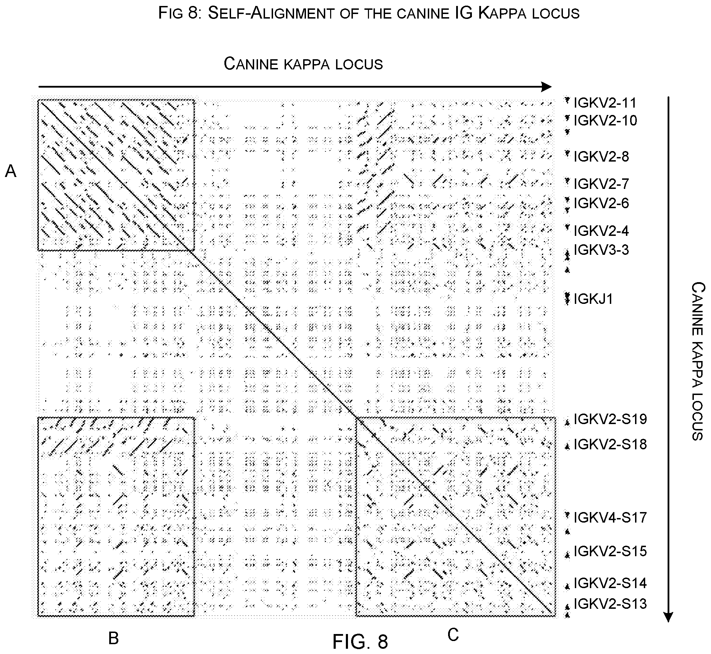

In the IGK self-alignment, three comparisons are of note: the upstream and downstream blocks' self-alignment **(****Figure 8** **green and red boxes, respectively),** as well as their alignment with each other **(****Figure 8** **blue box).** Within percentage identity plots (PIPs), lines indicate regions of identity, and so a sequence aligned against itself will always have a solid line running along the diagonal. Solid lines off the main diagonal indicate likely duplication events, with the gaps representing the indels and other mutations accumulated since the duplication.

The multiple broken diagonals in the upstream self-alignment is characteristic of a block, in this case a single V and its flanking sequence, being locally copied multiple times, similar to the block duplication of a three-gene cassette in the canine TCRB locus ¹⁶. In the self-alignment of the downstream block, the lines are shorter and at times perpendicular, implying local inverted homology. Finally, the homology comparison of the upstream to the downstream block reveals a good degree of homology, particularly IGKV2-S18 and IGKV-S19 to the IgKV2 genes upstream of the C gene. This pattern would be seen where a single gene replicated upstream of the C gene multiple times, then a block inversional duplication occurred in a manner reminiscent of human IGK and equine IGL. This downstream block was then likely under reduced selection pressure and has accumulated mutations and local inversions at a greater rate, with the exception of the IGKV2-S18 and IGKV-S19 genes, which have diverged less. This is not the only possible explanation, but it does fall in line with proposed explanations for similar features across other species and AR loci.

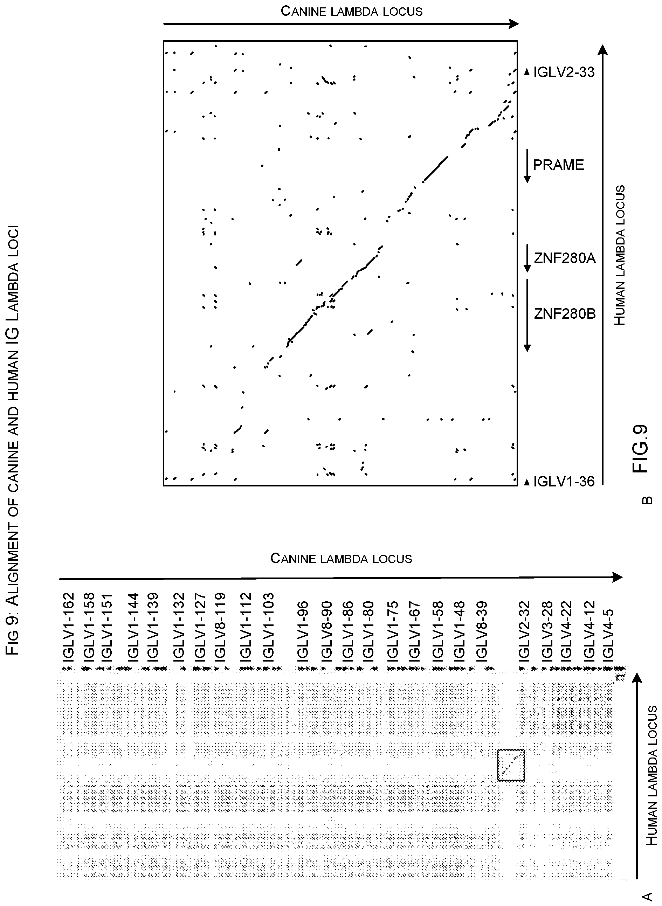

In the comparison of the dog and human IGL loci, two things are striking: the nearly unbroken diagonal near the midpoint of the human sequence, and the very high degree of homology characterised by the great number of lines at or near coding sequences (Figure 9a). Further analysis of the diagonal in the human/dog PIP reveals that it spans the region of the human locus that includes the non-AR genes ZNF280A, ZNF280B and PRAME (Figure 9b). The sequences for these genes, and most of the region surrounding them, are highly conserved between dog and human, implying that they are likely to be of similar functional importance in dog to humans. Specific comparisons show that the ZNF280B and PRAME genes are likely to be functional in the dog, but that the ZNF280A cannot be reliably identified at this locus (data not shown). Further investigations identify another non-IGL gene, PCBP2 near to ZNF280B.

Non-AR genes interspersed among the AR loci is a common feature across species, although the locations and genes in question are not always conserved. For example, ADAM6 genes are found in the IGH loci, in between the IGHV and IGHD genes of human and mouse, although the human orthologue is non-functional. Whilst no orthologue has been characterised in dog, there are two candidates. One of which is found between IGHV3-4 and IGHV3-5, and the other of which is upstream of the IGH locus entirely. Given the limited research into the ADAM gene family in dogs, this potential orthologue has not been added to the IGH annotation, but remains a candidate for future study. No other non-AR genes have been identified at this time in the canine IGK or IGL loci

### 3.1.5 Allele distribution

One of the defining characteristics of the use of dogs as a model organism is that whilst there is high inter-breed heterogeneity, within breed homogeneity is very high, to the point that it has been estimated that breed formation has accounted for a 35% loss in nucleotide diversity¹⁹. The selection has been so stringent that recent work identified 22 blocks of homozygosity longer than one megabase in certain breeds, which the authors attributed to breeder-imposed selection pressure²⁰ This contrasts with the diversity seen in humans. Even in geographically isolated populations, variation attributable to this separation is 5-10%, whereas more than one quarter of the genomic variation in dogs is attributable to breed, not individual, variation²¹.

Given the level of breed-specificity of canine genomics, it is perhaps surprising that the non-reference AR alleles did not appear to follow strong breed-specific haplotypes. The two best represented breeds in this sample were the Boxer and Standard Poodle (22 and 20 dogs, respectively) and they followed a similar pattern where a small number of non-reference alleles would be found relatively frequently in both breeds and the rarer alleles would be found on a single chromosome of one dog from one breed. The less well represented breeds followed similar distributions, and given the number of singularly represented alleles it seems that non-reference alleles are typically found in heterozygotes.

It is possible that larger breed-specific cohort sizes could reveal trends not apparent in this dataset, but as it stands it appears that the selective pressures that shape the AR gene loci are breed-agnostic and trump those of the breeds themselves. The likely exception would be in the case of evolutionary bottlenecks inflating the prevalence of less common allele. For example, IGLC1*01 (the reference allele) is found on 19 of the 44 Boxer chromosomes sequenced, and one of the six Toy Poodle chromosomes, but is not found in any other breeds. 194 of the 217 called alleles across all breeds are represented by the other allele, IGLC1*02. Whilst it is possible that the Toy Poodle has been outcrossed previously to boxers or that there was a sequencing error and this is indeed a Boxer-specific allele, it remains the case that a larger dataset is required to conclusively answer these questions one way or another.

Considering the distributions of the non-reference alleles, these all fall in line with what would be expected. That you see no change more often than expected is likely due to the genes in question having been subject to selective pressure for their current state, and so deviation from this is unfavourable. This could either be the loss of an effective functional gene, or the re-activation of an auto-reactive gene, in both cases the organism's fitness is reduced and so this would be selected against. Similarly, AR genes are very important to the fitness of an organism and their loss would come at a selection cost.

That gain of function is more common than expected lends weight to theories put forwards regarding the high pseudogene load of the AR loci. Pseudogene loads are generally high in AR loci, particularly in the dog, and are often expressed^{16,1,12,9}. They can gain functionality in recombination, for example where a stop codon is lost due to SHM or recombination itself, and act as a substrate for gene conversion ²². Assuming that the reference allele is the original, which is in line with their majority use, gain of function non-reference alleles are examples of the pseudogenes being a mutable starting pool for new beneficial alleles and are under a selection pressure as such.

### References

1. Das, S., Nozawa, M., Klein, J. & Nei, M. Evolutionary dynamics of the immunoglobulin heavy chain variable region genes in vertebrates. Immunogenetics 60, 47-55 (2008).
2. Olivieri, D., Faro, J., Von Haeften, B., Sánchez-Espinel, C. & Gambón-Deza, F. An automated algorithm for extracting functional immunologic V-genes from genomes in jawed vertebrates. Immunogenetics 65, 691-702 (2013).
3. Bao, Y., Guo, Y., Xiao, S. & Zhao, Z. Molecular characterization of the VH repertoire in Canis familiaris. Vet. Immunol. Immunopathol. 137, 64-75 (2010).
4. Lefranc, M. P. et al. IMGT®, the international ImMunoGeneTics information system®. Nucleic Acids Res. 37, 1006-1012 (2009).
5. Smit, A. F. A., Hubley, R. & Green, P. RepeatMasker. unpublished data Available at: http://www.repeatmasker.org/cgi-bin/WEBRepeatMasker.
6. Schwartz, S. PipMaker---A Web Server for Aligning Two Genomic DNA Sequences. Genome Res. 10, 577-586 (2000).
7. McWilliam, H. et al. Analysis Tool Web Services from the EMBL-EBI. Nucleic Acids Res. 41, W597-W600 (2013)
8. Letunic, I. & Bork, P. Interactive Tree Of Life (iTOL): an online tool for phylogenetic tree display and annotation. Bioinformatics 23, 127-128 (2007).
9. Hara, S., Diesterbeck, U. S., König, S. & Czerny, C. P. Transcriptional analysis of equine A-light chains in the horse breeds Rhenish-German Coldblood and Hanoverian Warmblood. Vet. Immunol. Immunopathol. 145, 50-65 (2012).
10. Schwartz, J. C., Lefranc, M.-P. & Murtaguh, M. P. Evolution of the porcine (Sus scrofa domestica) immunoglobulin kappa locus through germline gene conversion. Immunogenetics 64, 303-311 (2012)
11. Walther, S., Rusitzka, T. V, Diesterbeck, U. S. & Czerny, C. Equine immunoglobulins and organization of immunoglobulin genes. Dev. Comp. Immunol. 53, 303-319 (2015).
12. Kawasaki, K. et al. Evolutionary dynamics of the human immunoglobulin kappa locus and the germline repertoire of the Vkappa genes. Eur. J. Immunol. 31, 1017-28 (2001).
13. Schwartz, J. C., Lefranc, M.-P. & Murtaguh, M. P. Organization, complexity and allelic diversity of the porcine (Sus scrofa domestica) immunoglobulin lambda locus. Immunogenetics 64, 399-407 (2012)
14. Sun, Y., Wei, Z., Li, N. & Zhao, Y. A comparative overview of immunoglobulin genes and the generation of their diversity in tetrapods. Dev. Comp. Immunol. 39, 103-9 (2013).
15. Arun, S. S., Breuer, W. & Hermanns, W. Immunohistochemical examination of light-chain expression (lambda/kappa ratio) in canine, feline, equine, bovine and porcine plasma cells. Zentralbl. Veterinarmed. A 43, 573-6 (1996).
16. Mineccia, M. et al. New insight into the genomic structure of dog T cell receptor beta (TRB) locus inferred from expression analysis. Dev. Comp. Immunol. 37, 279-293 (2012).
17. Massari, S. et al. The deduced structure of the T cell receptor gamma locus in Canis lupus familiaris. Mol. Immunol. 46, 2728-2736 (2009).
18. Koop, B. F. & Hood, L. Striking sequence similarity over almost 100 kilobases of human and mouse T-cell receptor DNA. Nat Genet 7, 48-53 (1994).
19. Dobson, J. M. Breed-Predispositions to Cancer in Pedigree Dogs. ISRN Vet. Sci. 2013, 1-23 (2013).
20. Vaysse, A. et al. Identification of Genomic Regions Associated with Phenotypic Variation between Dog Breeds using Selection Mapping. PLoS Genet. 7, e1002316 (2011).
21. Parker, H. G. Genomic analyses of modern dog breeds. Mamm. Genome 23, 19-27 (2012).
22. Sun, Y. et al. Immunoglobulin genes and diversity: what we have learned from domestic animals. J. Anim. Sci. Biotechnol. 3, 18 (2012).

### Example 2 - Construction of chimaeric IG loci in murine cells

The IG loci of murine ES cells were modified by BAC insertion, to introduce canine heavy chain DNA into the murine IGH locus and canine light chain immunoglobulin DNA into the murine IGL kappa and lambda loci, as follows:

### Canine IGH insertion

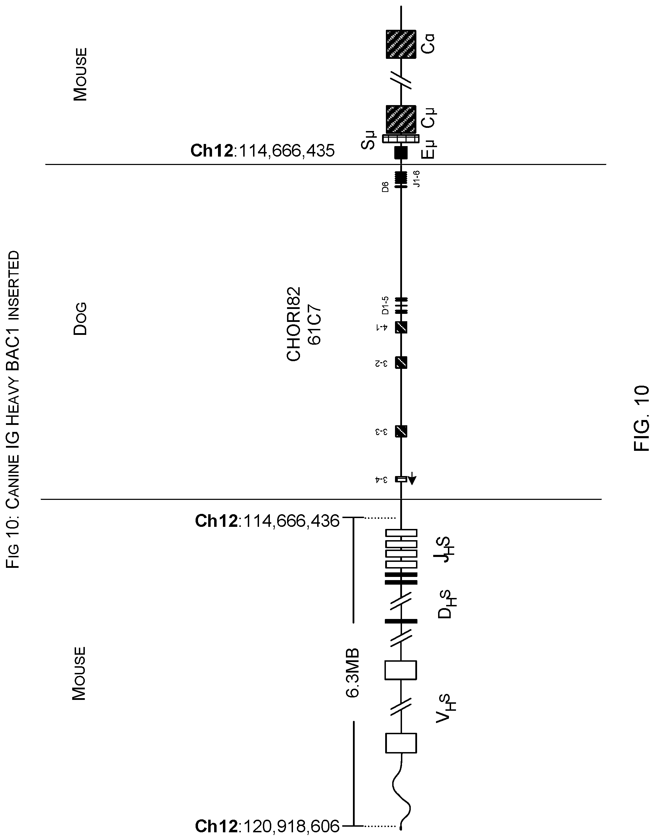

Insertion of canine DNA from chromosome 8 was made into the mouse IGH locus by BAC insertion. The inserted DNA comprised nucleotides 72,988,807-73,128,041 and contains IGHV4-1 to IGHV3-4, as well as IGHD1-6 and IGHJ1-6. See Figure 1 and 10.

### Canine IGL lambda DNA insertion

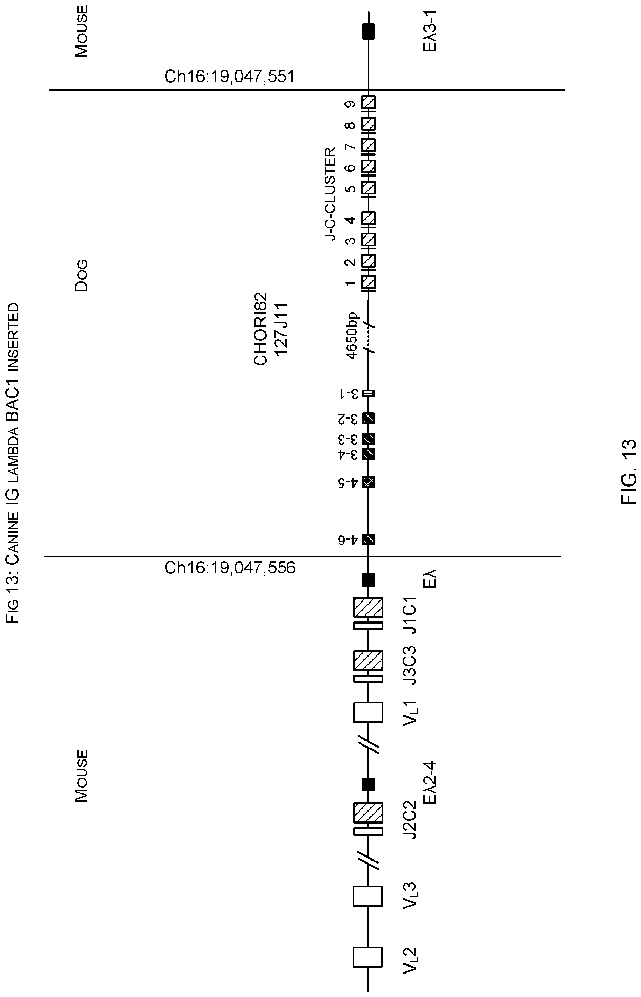

Insertion of canine DNA from chromosome 26 was made into the mouse IGL lambda locus on chromosome 16. The inserted DNA comprised nucleotides 27,509,860-27,646,373 and contains IGLV3-1 to IGLV4-6 as well as IGLJ1-9 and IGLC1-9. See Figures 2 and 13.

Co-ordinates are from the Dec 2011 GRCm38/mm10 assembly for the mouse and from Canfam3.1 for the dog.
Insertions of canine DNA were inserted into a landing pad located in the mouse genome at the following positions
Canine Heavy chain DNA: inserted immediately upstream of Mouse Chromosome 12, position 114,666435
Canine Kappa DNA: inserted immediately upstream of Mouse Chromosome 6: position 70,674,7.55
Canine Lambda DNA: inserted immediately upstream of Mouse Chromosome 16: position 19, 047, 551.

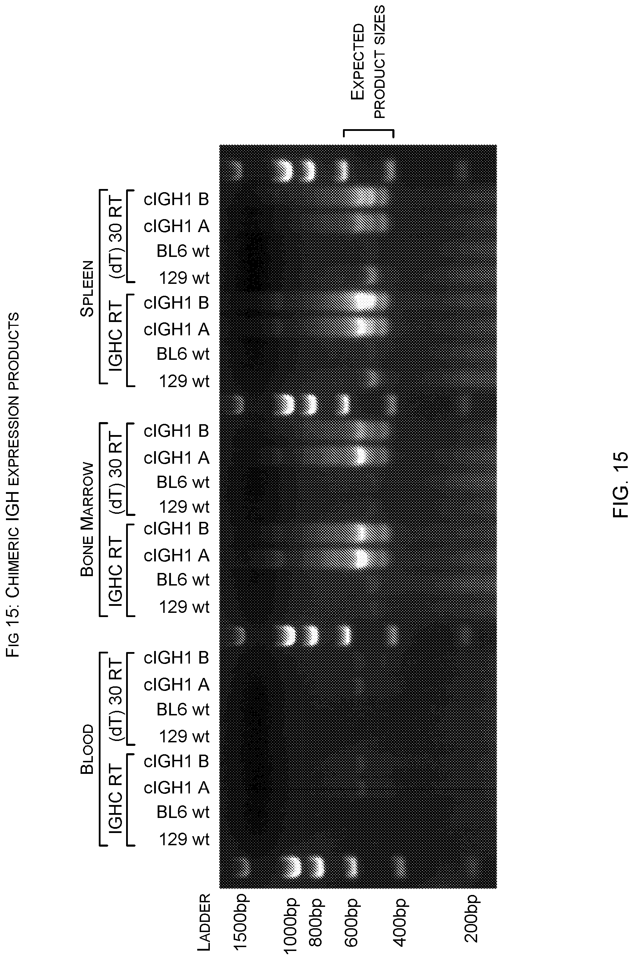

Expression of chimaeric transcripts has been confirmed by PCR analysis from the chimaeric heavy locus - see Figure 15.

### Methods were as follows:

### BAC modifications

Canine BACs derived from the CHORI-82 library were sourced directly from the BAC library of the Children's Hospital Oakland Research Institute.

All bacteria containing BACs were cultured at 32°C in Luria-Bertani (LB) broth or on LB-Agar, supplemented with 12.5ug/ml chloramphenicol. BAC-containing cells were rendered competent for recombination through the addition of the pSIM18 plasmid to the cells by a standard CaCl₂ heat-shock protocol, and maintenance of pSIM18 was selected for by supplementing media with 75 µg/ml hygromycin.

Recombineering plasmids, such as pRMCE38, were linearised and a 1kbp BAC-specific homology arm was introduced using Gibson assembly. The plasmids contain the sequences required for downstream recombineering, such as cre-lox and PiggbyBac sequences, as well as selection markers for BAC- or ESC-engineering steps. The finalised plasmids were restriction digested and gel-purified to yield a fragment spanning the BAC-specific homology arm, the recombineering sequences to be introduced, and the homology arm to the BAC's vector backbone. These fragments were inserted into the BACs by electroporation and successful integration of the fragments into the BAC was selected for.

Successful modification of resistant clones was verified by PCR across the junction of the inserted and endogenous DNA. Once both ends of the BAC were modified, BAC DNA was purified and electroporated into Electromax^{™} DH10B cells (Life Technologies). Clones that had lost hygromycin resistance, and therefore were pSIM18-negative, were selected for further analysis. PCR reactions were performed on the DNA from these clones to ensure the correct modification of either end, as well as the presence of the exons to be inserted using the relevant BAC. Clones that passed this quality control check were used for embryonic stem cell (ESC) engineering.

### ESC engineering

The process of ESC culture, electroporation, and drug selection were as described by Lee *et al.* 2014. Mouse male AB2.1 cells already containing landing pads for SRMCE at each of the immunoglobulin loci were used, and were cultured in M15 medium (knockout DMEM-supplemented with 15% FBS, 2 mM glutamine and 100 µM β-mercaptoethanol) and maintained on irradiated SNL76/7 feeders. All cells used were tested to ensure they were free of contaminants such as mycoplasma.

1 x 10⁷ cells were used for each transfection, and all transfections were carried out using a Bio-Rad electroporator (GenePulser Xcell) at 500 µF and 230 V. For the introduction of the BAC, 10ug of BAC DNA and 25ug of pCAGGS-iCRE was used per-transfection. Cells were selected in 3ug/ml puromycin after 24h for a week, and colonies were picked for expansion and testing. Successful incorporation of the BAC into the landing pad was verified with PCR using primers that spanned the junction of the mouse and BAC DNA.

Positive clones were then subjected to excision of the 3' landing pad using PBase. Clones were expanded and 1 x 10⁵ cells used per electroporation with 10ug PiggyBac transposase plasmid. After three days recovery in M15, cells were split and seeded at low density, before selection the next day with FIAU and maintained for ten days. Successful excision of the 3' end of the landing pad was confirmed by junction PCR. Positive clones were then subjected to the same exon tests as the original BACs to ensure no loss had occurred.

### Mouse generation and analysis

Positive ES cell clones were injected into blastocysts from the C57BL/6 Tyrc-Brd mouse strain by standard procedures. Injected blastocysts were transferred to the uteri of pseudopregnant female B6/CBA F1 recipients. Approximately 40 blastocysts were injected for each clone. High-percentage chimaeric males were mated with albino C57BL/6 Tyrc-Brd females, in order that pups derived from injected ESCs could be identified based on coat pigment. Mice derived from the ESC clone were subjected to PCR tests with primers that spanned the junction of the mouse and inserted DNA.

In order to verify the expression of inserted genes, a reverse-transcription-based approach was carried out. Blood, spleens, and femurs from mice confirmed to have the BAC present, as well as relevant wild-type controls, were harvested. The femurs were aspirated with PBS to yield a bone marrow sample. These three tissue types were converted into RNA using the NucleoSpin RNA-kit (Macherey-Nagel). Reverse transcription was carried out using superscript II (Thermo Fisher) using primers directed to the 5' end of the murine constant regions relevant to the inserted BAC (e.g. to the IGLCs for the mice containing the IGL BAC), or to the polyA tail of the mRNA. These transcripts were then amplified using barcoded primers nested to the murine C region, as well as those priming from the inserted leader regions from the canine V genes. This ensured that only chimaeric transcripts would be amplified, rather than all murine immunoglobulin transcripts. These PCR products were visualised on a gel, confirming the presence of chimeric transcripts in mice with the BAC, but not the wild-type mice. See figure 15 - confirmation of IGH chimaeric transcripts. The first 8 lanes correspond to RNA from blood samples, the second 8 to bone marrow samples, and the final 8 to spleen samples. The first four of a given 8 are C-region primed, the second four are primed from the polyA tail. Within the four, the first two are controls, and the latter two are mice expected to produce chimaeric transcripts.

All animal experimentation and husbandry was conducted under approval of the Wellcome Trust Sanger Institute AWERB (Animal Welfare and Ethics Review Body). United Kingdom Home Office approval is provided under Project License 80/2432.

### Example 3

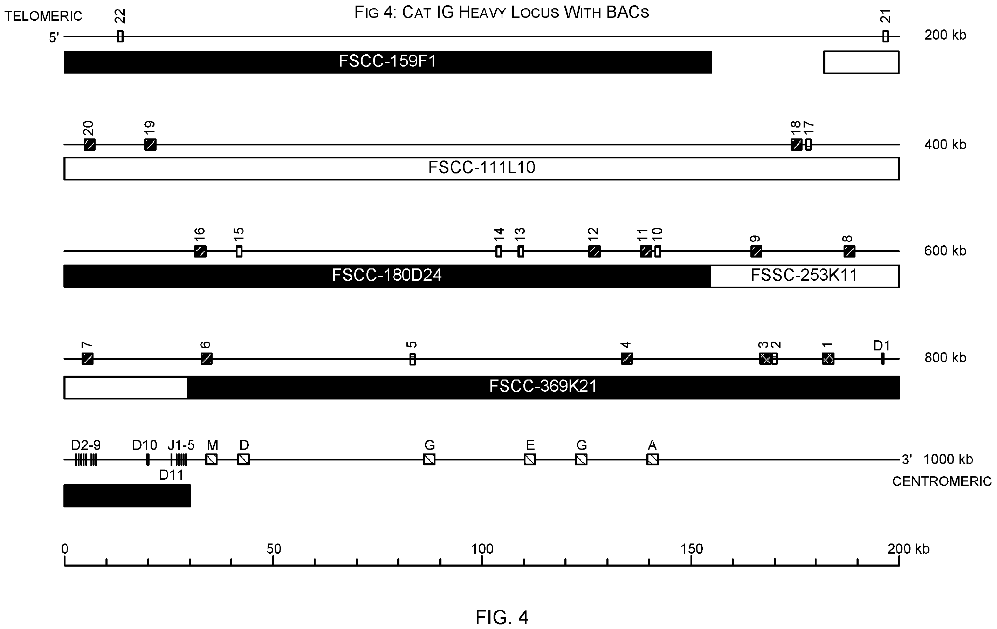

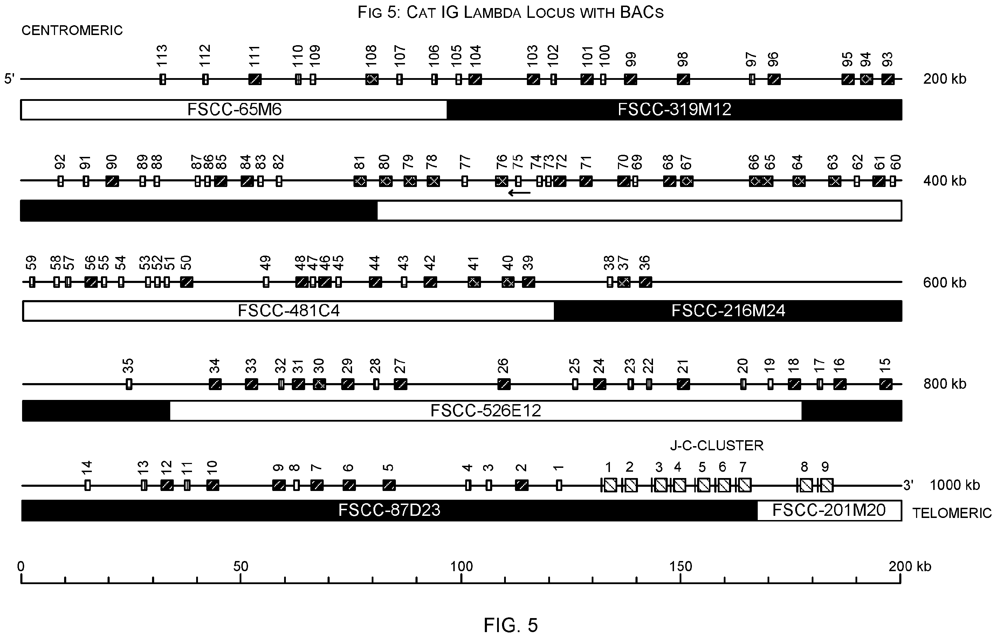

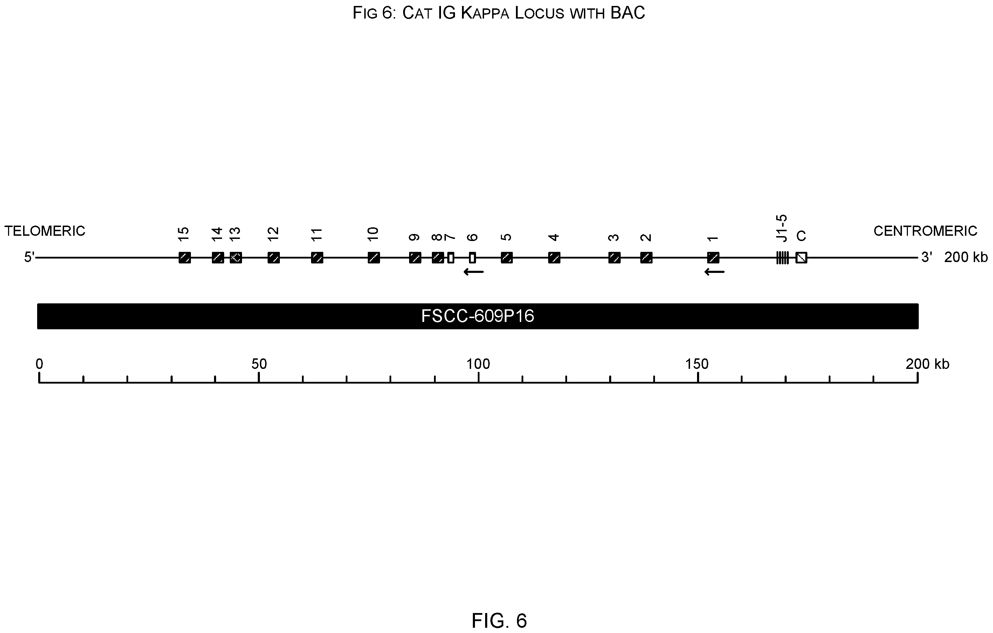

Figure 4, 5 and 6 show the annotation of the cat Ig loci. The method of annotation was the same as was outlined in 2.1.1 in Example 1, except that the cat genome was interrogated in place of the canine genome and no RNA-Seq data was used.

The annotation provides tools and information that allows for the use of cat DNA in the rodent genome, using methods as described above.

### Example 4 - 5' rapid amplification of cDNA ends (5'RACE)

### Materials and methods

### Blood samples

Baseline samples: Peripheral blood was drawn from thirteen dogs at the veterinary school of the University of Minnesota. RNA was extracted on site and shipped on dry ice. This study received prior approval from the ethics committee of the Veterinary School of the University of Minnesota.

### Library preparation

First strand cDNA was synthesised with a mixed pool of C-specific primers and the template switch oligo (TSO). The reaction contained: 666.7 µM dNTPs (Sigma Aldrich), 666.7 nM TSO, 333.3 nM of each of the heavy and light chain RT primer mixes, 1-5 µg RNA, 2 mM DTT (Invitrogen), 3 mM MgCl2, 40 units of RNaseOUT (Invitrogen), and 100 units of SuperScript II reverse transcriptase (Invitrogen) in a 30 µl volume. Extension was at 42°C for 60 minutes, after which 1 µl of RNase A/T1 mix (Thermo Scientific) was added and the reaction was incubated at 37°C for 15 minutes. The reaction was then cleaned up using the AMPure XP system (Agencourt) according to the manufactures recommendation (using an initial reaction:AMPure XP solution ratio of 5:4). The cDNA was resuspended in 21 µl of PCR-grade water and split evenly into one heavy and one light chain reaction. PCR was carried out in a volume of 25 µl using Q5 high fidelity polymerase (New England Biolabs), 10.5 µl of resuspended cDNA, and 400 nM each of 5'RFWA and either the heavy or light chain PCR1 mix. The cycling conditions were: 98°C 30 seconds, then 20 cycles of (98°C 10 seconds, 63°C 30 seconds, 72°C 20 seconds), followed by a final extension at 72°C for 2 minutes. The PCR reactions were subject to the same AMPure XP cleanup as before, and resuspended in 10.5 µl of PCR-grade water. This was made up to a 25 µL PCR reaction with Q5 polymerase, and 400 nM each of 5'RRVA and a forward primer containing a sample specific index hexamer. The PCR conditions were as before, and the product was cleaned up using the same AMPure XP system and resuspended in 20 µl PCR-grade water. These libraries were then quantified and pooled in an equimolar mix and diluted to a final concentration of 10 nM for sequencing.

### Sequencing and data analysis

Libraries were sequenced on a MiSeq machine (Illumina) using 300 bp paired-end reads (including 10% PhiX spike-in) by the core sequencing team at the Wellcome Trust Sanger Institute. The demultiplexed data was then filtered for quality prior to submission to the IMGT V-Quest software (using the 'HighV-Quest' software and selecting 'Species' = 'Canis lupus familiaris (dog)' and 'Receptor type or locus' = 'IG')¹⁴.

### Aspects of the invention are disclosed in the following numbered clauses:

1 A rodent or rodent cell having a genome comprising;
   i) one or more companion animal IGH V region genes, one or more companion animal D region genes and one or more companion animal J region genes; and
   ii) optionally one or more companion animal IGL kappa V region genes and one or more companion animal IGL kappa J region genes; and/or one or more companion animal IGL lambda V region genes and one or more companion animal IGL lambda J region genes,

   wherein the rodent or rodent cell is capable of expressing the companion animal variable region gene(s) to form an antibody chain
   and wherein the companion animal species is not a rodent.
2 A rodent or rodent cell having a genome comprising
   i) one or more companion animal IGL kappa V region genes and one or more companion animal IGL kappa J region genes; and/or one or more companion animal IGL lambda V region genes and one or more companion animal IGL lambda J region genes, and
   ii) optionally one or more companion animal IGH V region genes, one or more companion animal or host D region genes and one or more companion animal or host J region genes

   wherein the rodent or rodent cell is capable of expressing the companion animal variable region gene(s) to form an antibody chain
   and wherein the companion animal species is not a rodent.
3 The rodent or rodent cell of clause 1 or 2, wherein the one or more of the inserted companion animal V, D or J region genes is associated with a regulatory sequence from the same companion animal.
4 The rodent or rodent cell of clauses 1-3, wherein the rodent genome comprises companion animal genes from both the heavy chain and at least one light chain.
5 The rodent or rodent cell of any preceding clause comprising at least 4, 5, 10, 15, 20, 30, 40, 50, 60 70 or at least 80 companion animal IGH V region genes, optionally wherein the V region genes are canine.
6 The rodent or rodent cell of any preceding clause comprising at least 4, 5, 10, 15, 16, 17, 18 or 19 companion animal IGL kappa V region genes, optionally wherein the V region genes are canine.
7 The rodent or rodent cell of any preceding clause comprising at least 5, 10, 20, 30, 40, 50, 60, 70, 80, 90, 100, 110, 120, 130, 140, 150, or at least 160 companion animal IGL lambda V region genes, optionally wherein the V region genes are canine.
8 The rodent or rodent cell of any preceding clause wherein the companion animal gene(s) are located in the genome upstream of the rodent constant region, suitably upstream of the heavy chain constant region for inserted companion animal heavy chain variable region genes and suitably upstream of a light chain constant region for inserted companion animal light chain variable region genes, such that the rodent or rodent cell is able to produce a chimaeric antibody chain resulting from expression of the inserted variable region gene and the host constant region.
9 A rodent or rodent cell according to any one of clauses 1-7 having a genome in which the companion animal gene(s) are located into the genome in functional arrangement with a constant region from the same companion animal, such that the rodent is able to produce an antibody chain resulting from the expression of the inserted companion animal VDJ or VJ region genes and the companion animal constant region, optionally wherein the companion animal genes are lambda light chain V and J genes in functional arrangement with the lambda constant region from the same companion animal.
10 A rodent or rodent cell of any preceding clause comprising one or more companion animal IGL lambda V region genes, one or more companion animal IGL lambda J region genes and one or more companion animal lambda constant region genes located at the rodent cell kappa locus, such as downstream of the rodent kappa constant region.
11 A rodent or rodent cell of any preceding clause comprising canine kappa variable region genes, wherein all of the canine kappa variable region genes in the rodent genome are located upstream of constant region with which the variable region gene is expressed, optionally upstream of the host kappa constant region.
12 A rodent or rodent cell of any preceding clause wherein either all of the companion gene segments correspond to the canine reference alleles from CanFam 3.1, or wherein the companion gene segments comprise a non-reference allele from one or more of the following gene segments IGKV2-S13, IGLV 1-57, IGLV 1-68, IGLV 1-72, IGLV 1-88, IGLV 1-96, IGLV 8-60, IGLV 8-90 and IGLV 8-120.
13A rodent or rodent cell of any preceding clause wherein the rodent is a mouse and the companion animal is a dog, or a cat, or a horse, preferably a dog.
14 A method for producing a rodent or rodent cell according to any preceding clause, the method comprising inserting into a rodent cell genome one or more companion animal IGH V region genes, one or more companion animal IGH D region genes and one or more companion animal IGH J region genes, wherein the rodent or rodent cell is capable of expressing the companion animal variable region gene(s) in combination with a constant region to form an antibody chain.
15 A method for producing a rodent or rodent cell according to clause 14, the method comprising inserting into a rodent cell genome one or more companion animal IGL V region genes, and one or more companion animal IGL region J genes, wherein the rodent or rodent cell is capable of expressing the companion animal variable region gene(s) in combination with a constant region to form an antibody chain.
16 A method for producing antibody chain specific to a desired antigen, the method comprising immunizing a rodent according to any of clauses 1-13 with the desired antigen and recovering the antibody chain, singularly or as part of a complete antibody, or recovering a cell producing the antibody chain singularly or as part of a complete antibody.
17 A method for producing an antibody chain or antibody specific to a desired antigen and derived from a single species of companion animal, the method comprising immunizing a rodent comprising a companion genes according to any of clauses 1-13 and then replacing the rodent constant region of the antibody chain with a companion animal constant region from the same companion animal, suitably by engineering of the nucleic acid encoding the antibody.
18 A method for producing an antibody chain or part thereof, the antibody chain having a companion animal variable region, the method comprising expressing in a cell a DNA encoding the antibody chain, or a part thereof,
   wherein the sequence of the DNA encoding the variable region of the antibody chain is obtained from, or is obtainable by, immunising a rodent according to any one of clauses 1-13 with an antigen so that antibody chains are produced,
   optionally including the subsequent steps of:
      (i) purifying and /or isolating the antigen receptor chain, and
      (ii) optionally then formulating the antigen receptor chain into a pharmaceutically acceptable formulation suitable for administration into a companion animal.
19 An antibody or antibody chain, or part thereof, or a DNA encoding an antibody chain or a part thereof, which has been obtained or is obtainable from a rodent or rodent cell according to any one of clauses 1-13 or from a method of any one of clauses 16-18.
20 An antibody or antibody or chain, or part thereof, obtained or obtainable from a rodent or cell according to any one of clauses 1-13, for use in treatment or prevention of disease a companion animal in need thereof.
21 A method of treatment of a companion animal, the method comprising delivery of an antibody or antibody chain or part thereof to a companion animal in need thereof, the antibody being obtained or obtainable from a rodent or cell according to any one of clauses 1-13 or from a method of any one of clauses 16-18.
22 A method of replacing, in whole or part, in a rodent or rodent cell an endogenous immunoglobulin variable region gene locus with an homologous or orthologous companion animal gene locus and regulatory sequence, comprising:
   i) obtaining a cloned genomic fragment or synthetic sequence containing, in whole or in part, the homologous or orthologous companion animal gene locus and regulatory sequence;
   ii) using homologous recombination to genetically modify the cloned genomic fragment of (i) to create a large targeting vector for use in the rodent or rodent cell; and
   iii) introducing the vector of (ii) into the rodent or rodent cell to replace, in whole or part, the endogenous immunoglobulin variable gene locus.

## Claims

1. A mouse or mouse cell having a genome comprising:
i) one or more companion animal IGH V region genes, one or more companion animal D region genes and one or more companion animal J region genes; and
ii) optionally one or more companion animal IGL kappa V region genes and one or more companion animal IGL kappa J region genes; and/or one or more companion animal IGL lambda V region genes and one or more companion animal IGL lambda J region genes,
wherein the mouse or mouse cell is capable of expressing the companion animal variable region gene(s) to form an antibody chain in combination with all or part of an antibody constant region,
wherein the one or more of the inserted companion animal V, D or J region genes is associated with a regulatory sequence from the same companion animal, wherein the regulatory sequence directs the successful recombination of the V, D or J region genes,
wherein the companion animal species is a horse.

2. A mouse or mouse cell having a genome comprising:
i) one or more companion animal IGL kappa V region genes and one or more companion animal IGL kappa J region genes; and/or one or more companion animal IGL lambda V region genes and one or more companion animal IGL lambda J region genes, and
ii) optionally one or more companion animal IGH V region genes, one or more companion animal or host D region genes and one or more companion animal or host J region genes,
wherein the mouse or mouse cell is capable of expressing the companion animal variable region gene(s) to form an antibody chain in combination with all or part of an antibody constant region,
wherein the one or more of the inserted companion animal V, D or J region genes is associated with a regulatory sequence from the same companion animal, wherein the regulatory sequence directs the successful recombination of the V, D or J region genes,
wherein the companion animal species is a horse.

3. The mouse or mouse cell of claim 1 or 2, wherein the mouse genome comprises companion animal genes from both the heavy chain and at least one light chain.

4. The mouse or mouse cell of any preceding claim comprising at least 4, 5, 10, 15, 20, 30, 40, 50, 60 70 or at least 80 companion animal IGH V region genes, or at least 4, 5, 10, 15, 16, 17, 18 or 19 companion animal IGL kappa V region genes, or at least 5, 10, 20, 30, 40, 50, 60, 70, 80, 90, 100, 110, 120, 130, 140, 150, or at least 160 companion animal IGL lambda V region genes, optionally wherein the V region genes are equine.

5. The mouse or mouse cell of any preceding claim wherein the companion animal gene(s) are located in the genome, upstream of the mouse heavy chain constant region genes for inserted companion animal heavy chain variable region genes and upstream of a mouse light chain constant region gene(s) for inserted companion animal light chain variable region genes, such that the mouse or mouse cell is able to produce a chimaeric antibody chain resulting from expression of the inserted variable region gene and the host constant region.

6. A mouse or mouse cell according to any one of claims 1-4 having a genome in which the companion animal gene(s) are located into the genome in functional arrangement with a constant region from the same companion animal, such that the mouse is able to produce an antibody chain resulting from the expression of the inserted companion animal VDJ or VJ region genes and the companion animal constant region, optionally wherein the companion animal genes are lambda light chain V and J genes in functional arrangement with the lambda constant region from the same companion animal.

7. A mouse or mouse cell of any preceding claim comprising one or more companion animal IGL lambda V region genes, one or more companion animal IGL lambda J region genes and one or more companion animal lambda constant region genes located at the mouse cell kappa locus, within the locus or upstream or downstream of the mouse IGL kappa constant region.

8. A mouse or mouse cell of any preceding claim, comprising equine kappa variable region genes, wherein all the equine kappa variable region genes in the mouse genome are located upstream of constant region with which the variable region gene is expressed, optionally upstream of the host kappa constant region.

9. A mouse or mouse cell of any preceding claim, wherein all of the companion gene segments correspond to the equine reference alleles from the CHORI-241 BAC library .

10. A mouse or mouse cell of any preceding claim, wherein the regulatory sequence comprises a control sequence.

11. A mouse or mouse cell of any preceding claim, wherein the regulatory sequence comprises a recombination signal sequence (RSS).

12. A mouse or mouse cell of any preceding claim, comprising an RSS for V(D)J recombination.

13. A mouse or mouse cell of any preceding claim, wherein the one or more of the V, D or J region genes is flanked by an RSS sequence from the same animal.

14. A method for producing a mouse or mouse cell according to any preceding claim, the method comprising inserting into a mouse cell genome one or more companion animal IGH V region genes, one or more companion animal IGH D region genes and one or more companion animal IGH J region genes, wherein the one or more of the inserted companion animal V, D or J region genes is associated with a regulatory sequence from the same companion animal, wherein the regulatory sequence directs the successful recombination of the V, D or J region genes, wherein the mouse or mouse cell is capable of expressing the companion animal variable region gene(s) in combination with a constant region to form an antibody chain in combination with all or part of an antibody constant region,
wherein the companion animal is a horse.

15. A method for producing a mouse or mouse cell according to claim 14, the method comprising inserting into a mouse cell genome one or more companion animal IGL V region genes, and one or more companion animal IGL region J genes, wherein the mouse or mouse cell is capable of expressing the companion animal variable region gene(s) in combination with a constant region to form an antibody chain.

16. A method for producing antibody chain specific to a desired antigen, the method comprising immunizing a mouse according to any of claims 1-13 with the desired antigen and recovering the antibody chain, singularly or as part of a complete antibody, or recovering a cell producing the antibody chain singularly or as part of a complete antibody,
wherein the companion animal is a horse.

17. A method for producing an antibody chain or antibody specific to a desired antigen and derived from a single species of companion animal, the method comprising immunizing a mouse comprising a companion genes according to any of claims 1-13 and then replacing the mouse constant region of the antibody chain with a companion animal constant region from the same companion animal, suitably by engineering of the nucleic acid encoding the antibody,
wherein the companion animal is a horse.

18. A method for producing an antibody chain or part thereof, the antibody chain having a companion animal variable region, the method comprising expressing in a cell a DNA encoding the antibody chain, or a part thereof,
wherein the sequence of the DNA encoding the variable region of the antibody chain is obtained from, or is obtainable by, immunising a mouse according to any one of claims 1-13 with an antigen so that antibody chains are produced,
optionally including the subsequent steps of:
(i) purifying and /or isolating the antigen receptor chain, and
(ii) optionally then formulating the antigen receptor chain into a pharmaceutically acceptable formulation suitable for administration into a companion animal,
wherein the companion animal is a horse.
